# EUROPEAN PATENT APPLICATION

(11) **EP 4 428 797 A1**
(43) Date of publication of application: **11.09.2024**
(21) Application number: 22889939.9
(22) Date of filing: 31.10.2022
(51) Int. Cl.: G06Q 50/10, G06Q 50/22, G16H 20/30

(54) **CONTENT MANAGEMENT SERVER, CONTENT MANAGEMENT DEVICE, CONTENT MANAGEMENT SYSTEM, AND CONTENT MANAGEMENT PROGRAM**

(30) Priority: 04.11.2021 JP 2021180449; 24.06.2022 JP 2022101708; 19.07.2022 JP 2022114860
(71) Applicant: Niterra Co., Ltd., Nagoya-shi, Aichi 461-0005 (JP)
(72) Inventor: YAMADA Hidetoshi, Nagoya-shi, Aichi 461-0005 (JP); KOBA Yoshinobu, Nagoya-shi, Aichi 461-0005 (JP); KON Toshiki, Nagoya-shi, Aichi 461-0005 (JP); YOSHIMURA Ken, Nagoya-shi, Aichi 461-0005 (JP); IWATA Naohiro, Nagoya-shi, Aichi 461-0005 (JP); IKEDA Etsuya, Nagoya-shi, Aichi 461-0005 (JP); IZUTANI Chikao, Nagoya-shi, Aichi 461-0005 (JP); TAKAGI Yutaka, Nagoya-shi, Aichi 461-0005 (JP)
(74) Representative: Zimmermann & Partner Patentanwälte mbB
(86) International application number: PCT/JP2022/040757
(87) International publication number: WO 2023/080119

(57) **Abstract**

A content management server (20) includes a storage unit, a reception unit, and a transmission unit. The storage unit stores therein a first content that provides at least either a menu of a specific action or an effect of the specific action as information. The reception unit receives preparation action information about a preparation action before the specific action, from a first information terminal (30). The transmission unit transmits the first content to the first information terminal (30) in response to reception of the preparation action information.

## Description

### TECHNICAL FIELD

The present invention relates to a content management server, a content management device, a content management system, and a content management program.

### BACKGROUND ART

Patent Document 1 discloses a watching system including: an IC card carried by a target to be watched; a point device for exchanging points with an information device; and a watching server connected to the information device and the point device. When the IC card is touched to the point device by a person who is the target to be watched, the point device acquires points from the watching server and gives the points to the IC card. Such a configuration, in which the person who is the target to be watched touches the point device with the IC card when the person is outside the home, encourages the person to go out.

Patent Document 2 discloses a service system including: a user terminal and a service server for transmitting and receiving data to and from the user terminal. The user terminal transmits position information based on GPS information to the service server. The service server calculates the amount of exercise of a user on the basis of change of the position information. The service server provides a digital content (badge, coupon, or the like) to the user on the basis of the calculated amount of exercise. In this manner, the service system maintains and encourages activity of the user.

### PRIOR ART DOCUMENT

### PATENT DOCUMENT

Patent Document 1: Japanese Patent Application Laid-Open (kokai) No. 2012-150767
Patent Document 2: Japanese Patent Application Laid-Open (kokai) No. 2015-57693

### SUMMARY OF THE INVENTION

### PROBLEM TO BE SOLVED BY THE INVENTION

In each of the systems of Patent Documents 1 and 2, points or a digital content is acquired as a reward after a certain action such as going out. Accordingly, a motivation to perform the action is given to the target to be watched. However, there is room for improvement in these systems in terms of increasing a will to perform a specific action before performing the specific action.

An object of the present invention is to provide a technology capable of giving a motivation that easily increases a will to perform a specific action, before the user performs the specific action.

### MEANS FOR SOLVING THE PROBLEM

[1] A content management server for transmitting a content to a first information terminal used by a first user, the content management server including:
   a storage unit configured to store therein a first content that provides at least either a menu of a specific action or an effect of the specific action as information;
   a reception unit configured to receive preparation action information about a preparation action before the specific action, from the first information terminal; and
   a transmission unit configured to transmit the first content to the first information terminal in response to reception of the preparation action information.

The content management server in [1] can transmit the first content, which provides at least either the menu of the specific action or the effect of the specific action as information, to the first information terminal in response to reception of the preparation action information about the preparation action before the specific action. Therefore, the management server can give a motivation that easily increases the will to perform the specific action, to the first user who uses the first information terminal, at the stage of the preparation action before the specific action.

[2] The content management server described in [1], further including a determination unit in which a selection method for selecting the menu on the basis of vital information is defined in advance and which is configured to, when the reception unit receives vital information before the specific action of the first user, determine the menu on the basis of the vital information of the first user, wherein
the transmission unit transmits the first content including the menu determined by the determination unit, to the first information terminal.

Since the content management server in [2] can provide the menu based on the vital information of the first user before the specific action, the menu reflecting the status of the vitals of the first user before performing the specific action can be provided.

[3] The content management server described in [1] or [2], wherein, when the first information terminal transmits degree information indicating a degree of the specific action detected by the first information terminal, the reception unit receives the degree information.

Since the content management server in [3] can collect the "degree information indicating the degree of the specific action" detected by the first information terminal, the actual execution result of the first user can be properly grasped, and it is made possible to use the degree information later.

[4] The content management server described in [3], wherein, when the reception unit receives the degree information, the transmission unit transmits evaluation information or suggestion information reflecting the degree information, to the first information terminal.

The content management server in [4] enables evaluation and suggestion based on the result of the specific action actually performed by the first user who uses the first information terminal, so that a new motivation to perform the next specific action can be more effectively given to the first user.

[5] The content management server described in any one of [1] to [4], further including a handling unit configured to, when the reception unit receives vital information during or after the specific action of the first user, store the vital information in the storage unit and/or transmit the vital information to a second information terminal used by a second user who watches the first user.

The content management server in [5] can receive the vital information during or after the specific action of the first user, and it is made possible to use the vital information at the management server or the second information terminal. The vital information during or after the specific action is useful information for grasping what changes occurred in the body of the first user when the specific action was performed. Therefore, the content management server in [5] can give a motivation that easily increases the will of the first user, and also can make it easier to properly perform body management of the first user when the first user actually performs the specific action.

[6] The content management server described in [5], wherein
the handling unit transmits the vital information to the second information terminal, and
when response information corresponding to the vital information is transmitted from the second information terminal and the reception unit receives the response information, the transmission unit transmits the response information to the first information terminal.

When the first user performs the specific action, the content management server in [6] can provide, the vital information of the first user during or after the specific action to the second user side to allow the second user to make an evaluation, and can convey the response information returned from the second user side based on the vital information, to the first user side.

[7] The content management server described in any one of [1] to [6], wherein, when the preparation action information is received, the transmission unit transmits first notification information including information indicating that the specific action is to be started, to the second information terminal used by a second user who watches the first user.

The content management server in [7] allows the second user who watches the first user to confirm that the first user will start the specific action, on the basis of the information indicating that the specific action is to be started. Accordingly, the second user can confirm the safety of the first user before the specific action.

[8] The content management server described in any one of [1] to [7], wherein the reception unit receives the first content to be stored in the storage unit, from a second information terminal used by the second user who watches the first user.

The content management server in [8] allows the user of the second information terminal to decide on the content to be transmitted to the first user.

[9] The content management server described in any one of [1] to [8], wherein
the storage unit stores therein a second content in association with specific action information about the specific action, and
when it is determined that the first user has ended the specific action, the transmission unit transmits the second content to the first information terminal.

After the specific action, the content management server in [9] can transmit the second content to the first user who uses the first information terminal. Accordingly, the first user can recognize that the second content can be obtained after performing the specific action, so that a motivation to perform the specific action again can be given to the first user.

[10] The content management server described in any one of [1] to [9], wherein, when it is determined that the first user has ended the specific action, the transmission unit transmits second notification information including information indicating that the specific action has ended, to the second information terminal used by the second user who watches the first user.

The content management server in [10] allows the second user who watches the first user to confirm that the first user has ended the specific action, on the basis of the information indicating that the specific action has ended. Accordingly, the second user can confirm the safety of the first user after the specific action.

[11] The content management server described in [9] or [10] citing [9], wherein the reception unit receives the second content to be stored in the storage unit, from the second information terminal used by the second user who watches the first user.

The content management server in [11] allows the user of the second information terminal to decide on the details of the second content to be given to the first user after the specific action.

[12] A content management device for transmitting a content to a first information terminal used by a first user, the content management device including:
a storage unit configured to store therein a first content that provides at least either a menu of a specific action or an effect of the specific action as information;
a reception unit configured to receive preparation action information about a preparation action before the specific action, from the first information terminal; and
a transmission unit configured to transmit the first content to the first information terminal in response to reception of the preparation action information.

The content management device in [12] can transmit the first content, which provides at least either the menu of the specific action or the effect of the specific action as information, to the first information terminal in response to reception of the preparation action information about the preparation action before the specific action. Therefore, the management server can give a motivation that easily increases the will to perform the specific action, to the first user who uses the first information terminal, at the stage of the preparation action before the specific action.

[13] A content management device for outputting a content to a first user, the content management device including:
a storage unit configured to store therein a first content that provides at least either a menu of a specific action or an effect of the specific action as information;
a detection unit configured to detect a preparation action before the specific action; and
an output unit configured to output the first content directly or via another device when the preparation action is detected by the detection unit.

The content management device in [13] can transmit the first content, which provides at least either the menu of the specific action or the effect of the specific action as information, to the first information terminal in response to reception of the preparation action information about the preparation action before the specific action. Therefore, the management server can give a motivation that easily increases the will to perform the specific action, to the first user who uses the first information terminal, at the stage of the preparation action before the specific action.

[14] A content management system including: a first information terminal used by a first user; and a content management server configured to transmit a content to the first information terminal, wherein
the content management server includes
   a storage unit configured to store therein a first content that provides at least either a menu of a specific action or an effect of the specific action as information,
   a server-side reception unit configured to receive preparation action information about a preparation action before the specific action, from the first information terminal, and
   a server-side transmission unit configured to transmit the first content to the first information terminal in response to reception of the preparation action information, and
the first information terminal includes
   a first terminal-side transmission unit configured to transmit the preparation action information to the content management server,
   a first terminal-side reception unit configured to receive the first content from the content management server, and
   a first terminal-side output unit configured to output the first content received by the first terminal-side reception unit.

The content management system in [14] can transmit the first content, which provides at least either the menu of the specific action or the effect of the specific action as information, to the first information terminal in response to reception of the preparation action information about the preparation action before the specific action. Therefore, the management server can give a motivation that easily increases the will to perform the specific action, to the first user who uses the first information terminal, at the stage of the preparation action before the specific action.

[15] A content management program installed in a content management server configured to transmit a content to a first information terminal used by a first user, the content management program causing a computer to:
store therein a first content that provides at least either a menu of a specific action or an effect of the specific action as information;
receive preparation action information about a preparation action before the specific action, from the first information terminal; and
transmit the first content to the first information terminal in response to reception of the preparation action information.

The content management program in [15] can transmit the first content, which provides at least either the menu of the specific action or the effect of the specific action as information, to the first information terminal in response to reception of the preparation action information about the preparation action before the specific action. Therefore, the program can give a motivation that easily increases the will to perform the specific action, to the first user who uses the first information terminal, at the stage of the preparation action before the specific action.

[16] A content management program installed in a first information terminal used by a first user, the content management program causing a computer to:
transmit preparation action information about a preparation action before a specific action of the first user;
receive a first content that is associated with the preparation action information and that provides at least either a menu of a specific action or an effect of the specific action as information; and
output the received first content.

The content management program in [16] can cause the first information terminal to transmit the preparation action information about the preparation action before the specific action of the first user, and after the preparation action information is transmitted, when the "first content that provides at least either a menu of a specific action or an effect of the specific action as information" is transmitted and the first content is received, the content management program in [16] can cause the first information terminal to output the first content. Therefore, the program can give a motivation that easily increases the will to perform the specific action, to the first user who uses the first information terminal, at the stage of the preparation action before the specific action.

### ADVANTAGEOUS EFFECTS OF THE INVENTION

According to the present invention, a motivation that easily increases a will to perform a specific action can be given before a user performs the specific action.

### BRIEF DESCRIPTION OF THE DRAWINGS

Fig. 1 is an explanatory diagram schematically showing a content management system of a first embodiment.
Fig. 2 is a block diagram illustrating the electrical configuration of the content management system.
Fig. 3 is an explanatory diagram illustrating an outline of operation of the content management system before a specific action.
Fig. 4 is an explanatory diagram illustrating information stored by a content management server.
Fig. 5 is a flowchart showing the flow of action information transmission control executed by a first information terminal.
Fig. 6 is a flowchart showing the flow of first content transmission control executed by the content management server.
Fig. 7 is a flowchart showing the flow of first content output control executed by the first information terminal.
Fig. 8 is a flowchart showing the flow of notification control, based on first notification information, executed by a second information terminal.
Fig. 9 is an explanatory diagram illustrating an outline of operation of the content management system after the specific action.
Fig. 10 is a flowchart showing the flow of second content transmission control executed by the content management server.
Fig. 11 is a flowchart showing the flow of second content output control executed by the first information terminal.
Fig. 12 is a flowchart showing the flow of notification control, based on second notification information, executed by the second information terminal.
Fig. 13 is a block diagram illustrating the electrical configuration of a content management system of a second embodiment.
Fig. 14 is a block diagram illustrating the electrical configuration of a content management system of a third embodiment.
Fig. 15 is a flowchart showing the flow of content output control by a content management device of the third embodiment.
Fig. 16 is a block diagram illustrating the electrical configuration of a content management system of a fourth embodiment.
Fig. 17 is an explanatory diagram schematically showing a content management system of a fifth embodiment.
Fig. 18 is a block diagram illustrating the electrical configuration of the content management system of the fifth embodiment.
Fig. 19 is a flowchart showing the flow of content transmission control executed by a second information terminal of the fifth embodiment.
Fig. 20 is a flowchart showing the flow of content reception control executed by a first information terminal of the fifth embodiment.
Fig. 21 is an explanatory diagram illustrating an outline of operation of the content management system before a specific action of the fifth embodiment.
Fig. 22 is an explanatory diagram illustrating an outline of operation of the content management system after the specific action of the fifth embodiment.
Fig. 23 is a flowchart showing the flow of content output control executed by the first information terminal of the fifth embodiment.

### MODES FOR CARRYING OUT THE INVENTION

### 1. First embodiment

### 1-1. Configuration of content management system 10

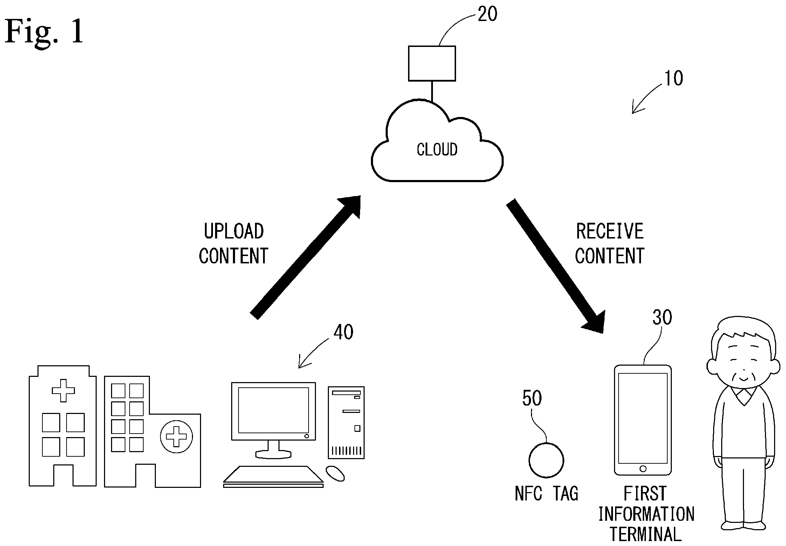

A content management system 10 shown in Fig. 1 includes a content management server 20, a first information terminal 30, a second information terminal 40, and an NFC (Near Field Communication) tag 50. The content management server 20 can communicate with the first information terminal 30 and the second information terminal 40. The first information terminal 30 is used by a first user who is assumed to perform a specific action. Specifically, the first user is a user who exercises, and is, for example, a user who is being treated for some disease, undergoing symptom relief, improving his/her health, or improving a part of his/her body. The first user may be a target to be watched by a second user, or may be a user to be managed or grasped by the second user. The specific action includes a rehabilitation program such as walking or running. The specific action may include another type of exercise such as cycling, and may include an exercise using exercise equipment such as a running machine or a cycling machine.

The second information terminal 40 is used by the second user. The second user is, for example, a user who manages or grasps treatment, improvement of health, or improvement of a part of the body of the first user. The second user may be a user who watches the first user. The content management system 10 is a system that can transmit a content uploaded from the second information terminal 40, to the first information terminal 30. In a typical example, the first user is a patient who is being treated for a disease, and the second user is a doctor who diagnoses, examines, and treats the patient, or may be a medical or nursing professional involved in the medical or nursing care of the patient. Examples of the content include photographs, moving images, sounds, etc. The content is configured as a reinforcer that increases a will of the first user to perform a specific action (walking, running, or the like), at a stage of a preparation action before the specific action. The content includes either one of or both a menu of the specific action and an effect obtained by performing the specific action.

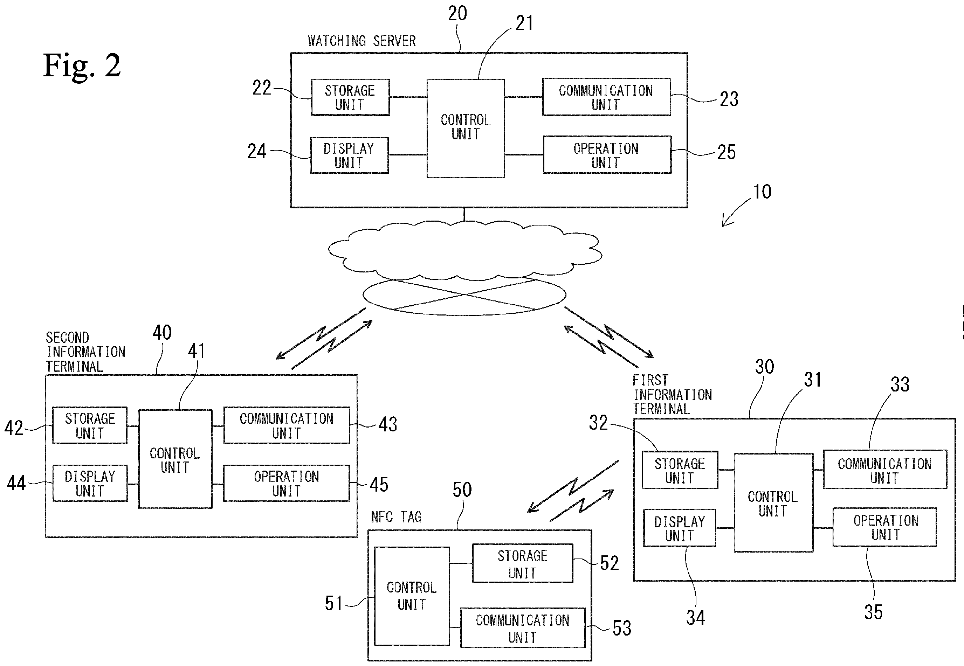

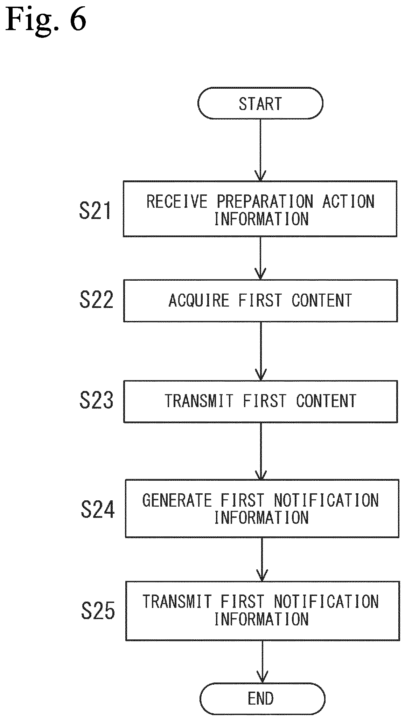

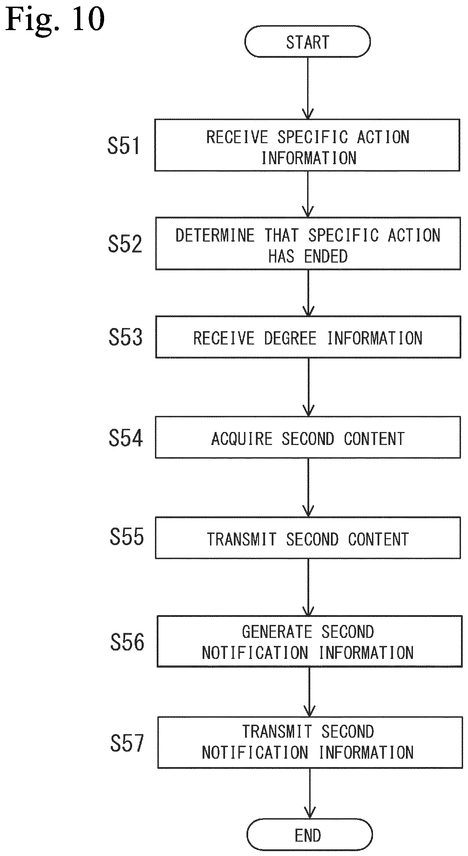

The content management server 20 is configured, for example, as a cloud server that can be used through a wide-area communication network (the Internet communication network or the like). As shown in Fig. 2, the content management server 20 includes a control unit 21, a storage unit 22, a communication unit 23, a display unit 24, and an operation unit 25. The control unit 21 controls the operation of the content management server 20. The control unit 21 is, for example, an information processing device mainly composed of a microcomputer, and has a CPU, etc. The storage unit 22 is composed of one or more known storage means such as a semiconductor memory. In the storage unit 22, a program for causing the control unit 21 to perform control (program that allows control described later (see Fig. 6 and Fig. 10) to be executed) is stored. The storage unit 22 stores therein contents described later. The communication unit 23 is configured as a communication module that can access the wide-area communication network (the Internet communication network or the like) using wireless communication. The communication unit 23 is configured to be able to wirelessly communicate with the first information terminal 30 and the second information terminal 40 via the wide-area communication network. The display unit 24 is configured as an image display device such as a liquid crystal display. The operation unit 25 is configured to allow information to be inputted by an operation of a user. The operation unit 25 is an input interface such as a keyboard and a touch panel.

The first information terminal 30 is configured as a portable information processing terminal, such as a smartphone, for example. As shown in Fig. 2, the first information terminal 30 includes a control unit 31, a storage unit 32, a communication unit 33, a display unit 34, and an operation unit 35. The control unit 31 is, for example, an information processing device mainly composed of a microcomputer, and has a CPU, etc. The storage unit 32 is composed of one or more known storage means such as a semiconductor memory. In the storage unit 32, a program for causing the control unit 31 to perform control (management application described later or the like), etc., are stored. The communication unit 33 is configured as a communication module that can access the wide-area communication network (the Internet communication network or the like) using wireless communication. The communication unit 33 is configured to be able to wirelessly communicate with the content management server 20 via the wide-area communication network. In addition, the communication unit 33 is configured as a communication module that can wirelessly communicate with the NFC tag 50 by an NFC communication method. The display unit 34 is, for example, a display unit that forms a part of a touch panel-type display device, and is configured as an image display device such as a liquid crystal display or an organic electroluminescent display. The operation unit 35 is configured, for example, as a known touch panel disposed so as to cover the front of the display unit 34, and is configured to cover the display part 34 in a transparent manner that allows an image on the display unit 34 to be viewed from the outside. The operation unit 35 may have any configuration as long as information can be inputted by an operation of a user, and may include an input interface (buttons, etc.) other than a touch panel.

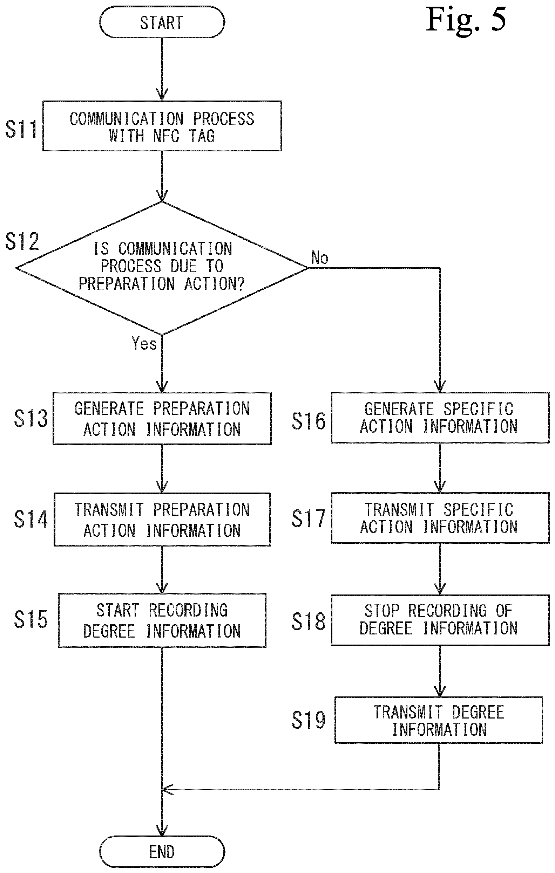

In the first information terminal 30, an application program for communication with the content management server 20 (hereinafter, also referred to as management application) is installed. The first information terminal 30 performs communication with the content management server 20, for example, in a state where the application program is executed (i.e., a state where the application has been launched). The application program installed in the first information terminal 30 is a program that allows control described later (see Fig. 5, Fig. 7, and Fig. 11) to be executed. The application program may be executed in the background or in the foreground at the first information terminal 30.

The second information terminal 40 is configured as a portable information processing terminal, such as a personal computer or a smartphone, for example. As shown in Fig. 2, the second information terminal 40 includes a control unit 41, a storage unit 42, a communication unit 43, a display unit 44, and an operation unit 45. The control unit 41, the storage unit 42, the communication unit 43, the display unit 44, and the operation unit 45 have the same configurations as the control unit 21, the storage unit 22, the communication unit 23, the display unit 24, and the operation unit 25 of the first information terminal 30, respectively. The second information terminal 40 is, for example, a terminal installed in a medical institution or the like, and is, for example, a terminal used by a doctor, a medical professional, or the like as the second user.

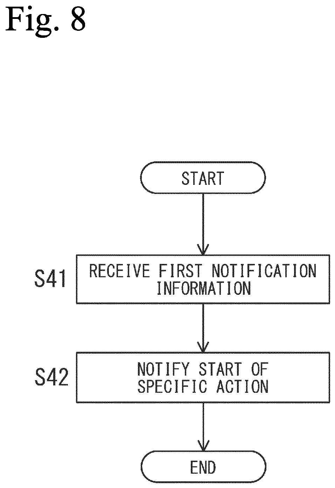

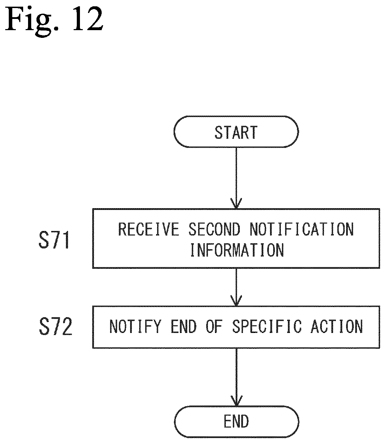

In the second information terminal 40, an application program for communication with the content management server 20 is installed. The second information terminal 40 performs communication with the content management server 20, for example, in a state where the application program is executed (i.e., a state where the application has been launched). The application program installed in the second information terminal 40 is a program that allows control described later (see Fig. 8 and Fig. 12) to be executed. The application program may be executed in the background or in the foreground at the second information terminal 40.

The NFC tag 50 can perform wireless communication with the first information terminal 30 by the NFC communication method. The NFC tag 50 corresponds to an example of a "wireless tag". As shown in Fig. 2, the NFC tag 50 includes a control unit 51, a storage unit 52, and a communication unit 53. The control unit 51 is, for example, an information processing device mainly composed of a microcomputer, and has a CPU, etc. The storage unit 52 is composed of one or more memories such as a semiconductor memory. In the storage unit 52, unique information for identifying the NFC tag 50, etc., are stored. The communication unit 53 has, for example, an antenna, a power supply circuit, a demodulation circuit, a modulation circuit, which are not shown, etc. The power supply circuit rectifies and smooths a reception signal (carrier signal) received via the antenna to generate an operation power supply, and supplies the operation power supply to each component such as the control unit 51. The demodulation circuit demodulates data superimposed on the reception signal (carrier signal) and outputs the data to the control unit. The control unit 51 is configured to read the above component information, etc., from the storage unit 52 and output the information, etc., as transmission data to the modulation circuit. The modulation circuit is configured to perform load modulation of a response signal (carrier signal) with the transmission data and transmit the resultant signal from the antenna. The above-described configuration of the NFC tag 50 and the configuration in Fig. 2 are merely examples, and other known configurations may be adopted as long as the wireless tag is capable of reading information by a wireless communication method.

### 1-2. Outline of operation of content management system

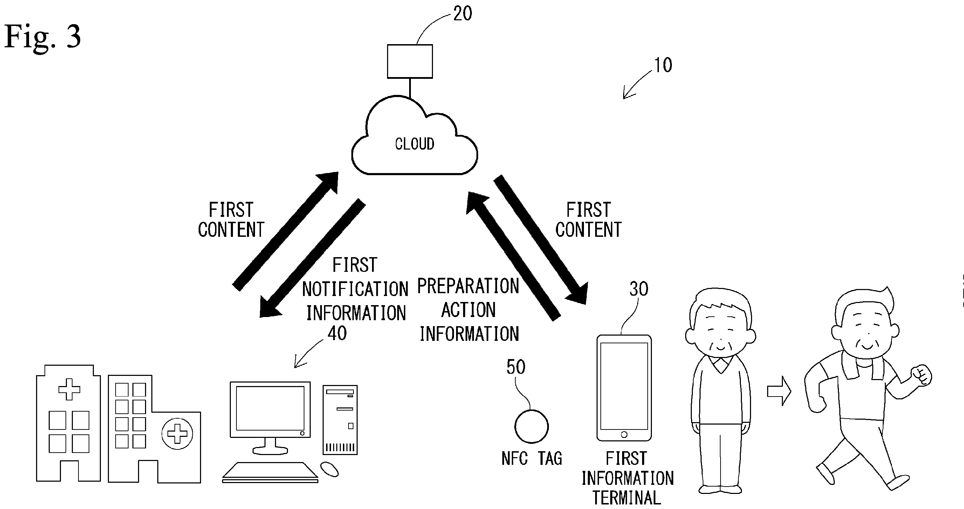

Fig. 3 is an explanatory diagram illustrating an outline of operation of the content management system before the specific action. When preparation action information described later is transmitted from the first information terminal 30 to the content management server 20, a first content uploaded from the second information terminal 40 to the content management server 20 is transmitted to the first information terminal 30. Furthermore, when the preparation action information described later is transmitted from the first information terminal 30 to the content management server 20, first notification information described later is transmitted from the content management server 20 to the second information terminal 40.

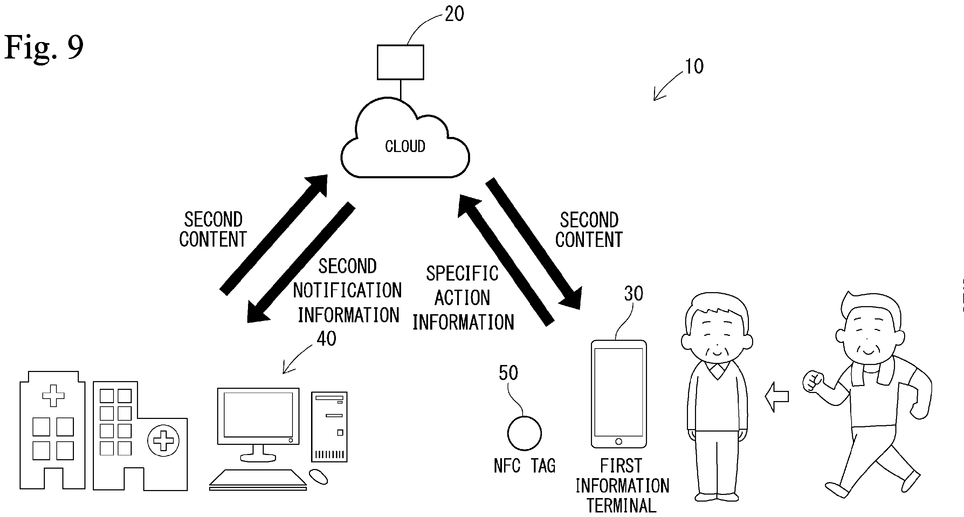

Fig. 9 is an explanatory diagram illustrating an outline of operation of the content management system after the specific action. When specific action information described later is transmitted from the first information terminal 30 to the content management server 20, a second content uploaded from the second information terminal 40 to the content management server 20 is transmitted to the first information terminal 30. Furthermore, when the specific action information described later is transmitted from the first information terminal 30 to the content management server 20, second notification information described later is transmitted from the content management server 20 to the second information terminal 40.

### 1-3. Upload of content

A content is uploaded to the content management server 20 by the second information terminal 40 in advance. The second information terminal 40 transmits a content to the content management server 20, for example, in a state where the management application has been launched. For example, a content stored in advance in the second information terminal 40 is transmitted. The content management server 20 stores the content received from the second information terminal 40, in the storage unit 22. Accordingly, the content is uploaded in the content management server 20. The control unit 21 and the communication unit 23 correspond to an example of a "reception unit" of the present invention, and receive the first content and the second content from the second information terminal 40. The first content is a content transmitted to the first information terminal 30 before the specific action. The second content is a content transmitted to the first information terminal 30 after the specific action. The storage unit 22 corresponds to an example of a "storage unit" of the present invention.

The first content and the second content are contents that include useful information for the first user, and are contents that include information such as the menu of the specific action and the effect obtained when the specific action is performed. The first content is a reinforcer that increases the will to perform the specific action at the stage of the preparation action before the specific action. The second content is a reinforcer that increases a will to perform the next specific action at the stage of end of the specific action.

In a typical example described below, the first user is a user with a specific type of disease. The first content is a content that can output information, such as a menu of an exercise suitable for the above specific type of disease and an effect obtained by performing the exercise suitable for the above specific type of disease, in the form of still images, moving images, sounds, etc. The second content may include, as information, the next exercise menu suitable for the above specific type of disease, an effect obtained by the most recently completed current exercise, etc.

In the content management server 20, various contents are stored in association with action information of the first user. The action information is information for specifying actions of the first user (preparation action information and specific action information described later). The action information may be, for example, character string data for specifying the actions, or may be data such as numbers or symbols. The specific action is an action to be reinforced (action to be prompted) for the first user, and is, for example, walking, running, other exercise, or the like. The specific action information is information about the specific action, and is information indicating that the specific action has been performed. The preparation action is an action before performing the specific action, and is, for example, an action of bringing the NFC tag 50 close to the first information terminal 30 in a state where the management application has been launched, or the like. The preparation action information is information about the preparation action, and is information indicating that the preparation action has been performed.

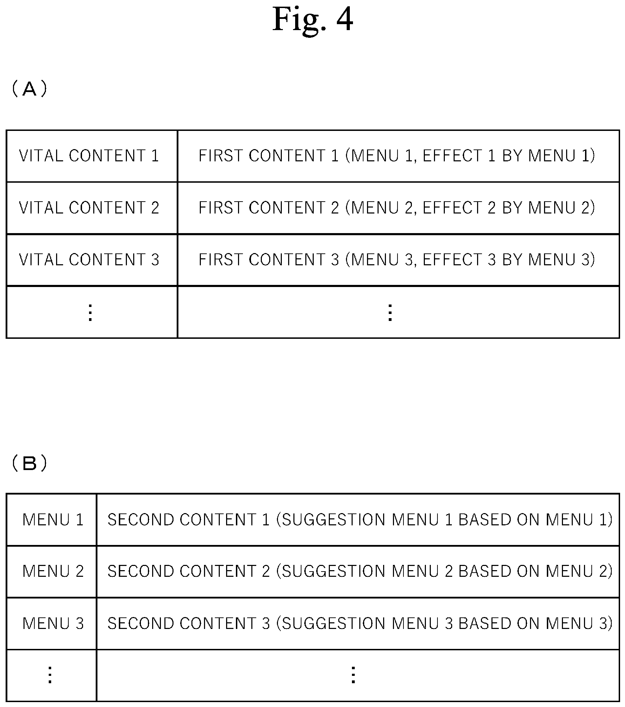

For example, as shown in Fig. 4(A), in the content management server 20, candidates of information to be provided are stored in association with the preparation action information. The candidates of information to be provided are classified into multiple types of vital contents, and each first content is stored in association with each vital content. Furthermore, in an example in Fig. 4(B), a second content is defined in association with each menu.

### 1-4. Action information transmission control by first information terminal 30

Action information transmission control by the first information terminal 30 will be described with reference to a flowchart shown in Fig. 5. The flowchart shown in Fig. 5 is a flowchart showing the flow of the action information transmission control executed by the control unit 31 of the first information terminal 30.

The control unit 31 performs the control in Fig. 5, for example, when communication between the first information terminal 30 and the NFC tag 50 becomes possible. When performing the control in Fig. 5, first, the control unit 31 performs a communication process with the NFC tag 50 (step S11). For example, the first user brings the NFC tag 50 close to the first information terminal 30 in a state where the management application has been launched by the first information terminal 30. As a result, the communication process is performed between the first information terminal 30 and the NFC tag 50. For example, the NFC tag 50 starts operation on the basis of radio waves generated by the first information terminal 30, and data (request data, response data, etc.) is exchanged between the first information terminal 30 and the NFC tag 50.

Subsequently, the control unit 31 determines whether or not the communication process performed in step S11 is due to the preparation action before the specific action (step S12). For example, when recording of data of action (hereinafter, also referred to as degree information) has not been performed by the management application before the communication process between the first information terminal 30 and the NFC tag 50, the control unit 31 determines that the communication process is due to the preparation action. The degree information is data such as numerical values that specify a movement distance, the number of steps taken, etc. When the control unit 31 determines that the communication process performed in step S11 is due to the preparation action before the specific action, the control unit 31 proceeds to Yes and generates preparation action information (step S13). The preparation action information is, for example, information indicating that the communication process has been performed between the first information terminal 30 and the NFC tag 50.

The preparation action information preferably includes vital information of the first user before the specific action is performed (e.g., when the preparation action is completed). Examples of the vital information include a blood pressure, a heart rate, an electrocardiogram, a respiration rate, SpO₂, etc., of the first user. The vital information of the first user may be measured by a measurement device provided in the first information terminal 30, or the first information terminal 30 may acquire values measured by another measurement device different from the first information terminal 30, via wireless communication.

Subsequently, the control unit 31 transmits the preparation action information generated in step S13, to the content management server 20 (step S14). The control unit 31 and the communication unit 33 correspond to an example of a "first terminal-side transmission unit", and transmit the preparation action information to the content management server 20 when wireless communication is performed with the NFC tag 50. Thereafter, the control unit 31 starts recording data (degree information) of the specific action (step S15). For example, if the specific action is walking or running, a movement distance, the number of steps taken, etc., correspond to the degree information. The distance and the number of steps taken can be measured, for example, by the operation of a GPS device and a pedometer included in the first information terminal 30. After step S15, the control unit 31 ends the action information transmission control shown in Fig. 5.

On the other hand, when the control unit 31 determines in step S12 that the communication process performed in step S11 is not due to the preparation action before the specific action (but is due to an action after the specific action), the control unit 31 proceeds to No and generates specific action information (step S16). The specific action information is, for example, information indicating that the communication process was performed by the first information terminal 30 and the NFC tag 50 after the specific action was performed. Whether or not the communication process was performed by the first information terminal 30 and the NFC tag 50 after the specific action was performed, can be determined, for example, on the basis of degree information (a movement distance, the number of steps taken, etc.) recorded before the communication process and after a previous communication process. In this case, when the movement distance or the number of steps taken does not reach a certain degree, it can be determined that the preparation action has been performed. When the movement distance or the number of steps taken reaches the certain degree, it can be determined that the specific action, not the preparation action, has been performed.

Subsequently, the control unit 31 transmits the specific action information generated in step S16, to the content management server 20 (step S17). Thereafter, the control unit 31 stops the recording of the degree information of the specific action that has been started in step S15 previously performed (step S18). Thereafter, the control unit 31 transmits the degree information whose recording has been stopped in step S18, to the content management server 20 (step S19). The control may be performed such that the specific action information and the degree information are simultaneously transmitted to the content management server 20 after the recording of the degree information is stopped. After step S19, the control unit 31 ends the action information transmission control shown in Fig. 5.

### 1-5. First content transmission control by content management server 20

First content transmission control by the content management server 20 will be described with reference to a flowchart shown in Fig. 6. The flowchart shown in Fig. 6 is a flowchart showing the flow of the first content transmission control executed by the control unit 21 of the content management server 20.

The control unit 21 executes the control in Fig. 6, for example, when the preparation action information is transmitted from the first information terminal 30. When executing the control in Fig. 6, first, the control unit 21 receives the preparation action information transmitted from the first information terminal 30 in step S14 (step S21). The control unit 21 and the communication unit 23 correspond to examples of the "reception unit" and a "server-side reception unit" of the present invention. Subsequently, the control unit 21 acquires the first content stored in association with the preparation action information received in step S21, from the storage unit 22 (step S22). For example, as shown in Fig. 4(A), among the candidate information associated with the preparation action information, the first content associated with a vital content that matches the vital information of the first user is read from the storage unit 22.

The vital content may be, for example, a vital content that defines a blood pressure range. For example, as Vital Content 1 shown in Fig. 4(A), a first blood pressure range which is relatively high can be defined, and a "first content 1 including a menu that suggests a light exercise such as stretching" can be prepared in association with Vital Content 1. As Vital Content 2, a second blood pressure range which is lower than the first blood pressure range can be defined, and a "first content 2 including a menu that suggests an exercise whose load is higher than that of stretching, such as walking" can be prepared in association with Vital Content 2. As Vital Content 3, a third blood pressure range which is lower than the second blood pressure range can be defined, and a "first content 3 including a menu that suggests an exercise whose load is higher than that of walking, such as jogging" can be prepared in association with Vital Content 3. In this case, if the blood pressure of the first user is within the first blood pressure range, the above first content 1 is read.

As another example, the vital content may be, for example, a vital content that defines SpO₂. For example, as Vital Content 1, "SpO₂ is equal to or lower than a reference value" can be defined, and a "first content 1 including a menu that suggests a light exercise such as stretching" can be prepared in association with Vital Content 1. As Vital Content 2, "SpO₂ exceeds the reference value" can be defined, and a "first content 2 including a menu that suggests an exercise whose load is higher than that of stretching, such as walking" can be prepared in association with Vital Content 2. In this case, if SpO₂ of the first user is equal to or lower than the reference value, the above first content 1 is read.

The correspondence between the vital content and the menu is merely an example. Even when one of the above conditions is satisfied, if the heart rate and the electrocardiogram are irregular or if arrhythmia is detected, a first content including a light exercise menu such as stretching or a first content recommending having a medical examination at a hospital may be selected. Alternatively, if the respiration rate is higher than a reference value for the first user, a first content including a light exercise menu such as stretching or a short walking route may be selected.

Each first content may include information of an effect obtained by the suggestion menu included in each first content. For example, if the menu included in the first content is a menu including a light exercise such as upper limb stretching, information of an effect that "stiffness of the respiratory muscles is relaxed, which reduces difficulty in breathing and makes breathing easier" can be included in association with this menu. Alternatively, if the menu included in the first content is a menu including walking such as strolling, information of an effect that "exercise such as strolling increases the muscle mass of the whole body including the lower limbs, which makes exercise easier and reduces breathlessness or difficulty in breathing during exercise" can be included in the menu.

Subsequently, the control unit 21 transmits the first content acquired in step S22, to the first information terminal 30 (step S23). The control unit 21 and the communication unit 23 correspond to examples of a "transmission unit" and a "server-side transmission unit", and transmit the first content to the first information terminal 30 in response to the reception of the preparation action information. In the present embodiment, the control unit 21 corresponds to an example of a determination unit, and functions to determine a menu (specifically, a content including a menu) on the basis of the vital information of the first user when the reception unit receives the vital information of the first user before the specific action. The transmission unit functions to transmit the first content including the menu determined by the determination unit to the first information terminal 30.

Subsequently, the control unit 21 generates first notification information (step S24). The first notification information is information including information indicating that the specific action is to be started. Specifically, the first notification information is information that notifies that the first user is about to start walking or running. Subsequently, the control unit 21 transmits the first notification information generated in step S24, to the second information terminal 40 (step S25). The control unit 21 and the communication unit 23 correspond to an example of the "transmission unit", and transmit the first notification information including information indicating that the specific action is to be started, to the second information terminal 40 when the preparation action information is received. After step S25, the control unit 21 ends the first content transmission control shown in Fig. 6.

### 1-6. First content output control by first information terminal 30

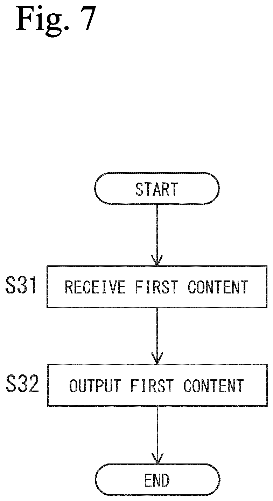

First content output control by the first information terminal 30 will be described with reference to a flowchart shown in Fig. 7. The flowchart shown in Fig. 7 is a flowchart showing the flow of the first content output control executed by the control unit 31 of the first information terminal 30.

The control unit 31 performs the control in Fig. 7, for example, when the first content is transmitted from the content management server 20 to the first information terminal 30. When performing the control in Fig. 7, first, the control unit 31 receives the first content transmitted from the content management server 20 in step S23 (step S31). The control unit 31 and the communication unit 33 correspond to an example of a "first terminal-side reception unit". Subsequently, the control unit 31 outputs the first content (content for image display and sound output of the menu and effect described above) received in step S31 (step S32). The control unit 31 and the display unit 34 correspond to an example of a "first terminal-side output unit". For example, if the first content is a still image, the control unit 31 causes the display unit 34 to display the first content as a still image thereon. If the first content is a moving image, the control unit 31 causes the display unit 34 to display the first content as a moving image thereon. If the first content is a sound, the control unit 31 causes the first content to be presented as a sound by a speaker or the like additionally provided in the first information terminal 30. The first content may have any configuration, such as a combination of a moving image and a sound, as long as information is outputted.

By outputting the first content by the first information terminal 30, a reinforcer (first content) that increases the will of the first user to perform the specific action, at the stage of the preparation action before the specific action, is given to the first user at the stage of the preparation action before the specific action. Accordingly, a motivation to perform the specific action can be given to the first user. In addition, since the specific action of the first user is immediately reinforced, the specific action can be continued and a habit of the specific action can be acquired. If the specific action is an exercise suitable for the above specific type of disease, an effect of improving or inhibiting the worsening of the symptoms of the above specific disease is obtained. After step S32, the control unit 31 ends the first content output control shown in Fig. 7.

### 1-7. Notification control, based on first notification information, by second information terminal 40

Notification control, based on the first notification information, by the second information terminal 40 will be described with reference to a flowchart shown in Fig. 8. The flowchart shown in Fig. 8 is a flowchart showing the flow of the notification control, based on the first notification information, executed by the control unit 41 of the second information terminal 40.

The control unit 41 performs the control in Fig. 8, for example, when the first notification information is transmitted from the content management server 20. When performing the control in Fig. 8, first, the control unit 41 receives the first notification information transmitted from the content management server 20 in step S25 (step S41). The control unit 41 and the communication unit 43 correspond to an example of a "second terminal-side reception unit". Subsequently, the control unit 41 notifies the start of the specific action on the basis of the first notification information received in step S41 (step S42). The control unit 41 and the display unit 44 correspond to an example of a "second terminal-side notification unit", and perform notification on the basis of the received first notification information. For example, a pop-up window is displayed on the display unit 44 of the second information terminal 40 to indicate that the specific action is to be started. More specifically, a message such as "Walking has started" may be displayed on the display unit 44. Alternatively, an e-mail may be received from the content management server 20 in step S41, and information indicating that the specific action is to be started may be included in the e-mail as a content thereof.

Through the notification based on the first notification information, the second user (user of the second information terminal 40) can confirm the safety of the first user before the specific action. In addition, the second user can not only receive the first notification information (information about the safety of the first user before the specific action) but also can decide on the first content to be transmitted to the first user, and thus a kind of communication can be performed. Communication between the first user and the second user can be prompted by the event or topic derived from the first content. After step S42, the control unit 41 ends the notification control, based on the first notification information, shown in Fig. 8.

### 1-8. Second content transmission control by content management server 20

Second content transmission control by the content management server 20 will be described with reference to a flowchart shown in Fig. 10. The flowchart shown in Fig. 10 is a flowchart showing the flow of the second content transmission control executed by the control unit 21 of the content management server 20.

The control unit 21 performs the control in Fig. 10, for example, when the specific action information is transmitted from the first information terminal 30. When performing the control in Fig. 10, first, the control unit 21 receives the specific action information transmitted from the first information terminal 30 in step S17 (step S51). Subsequently, the control unit 21 determines that the specific action of the first user has been ended (step S52). Subsequently, the control unit 21 receives the degree information transmitted from the first information terminal 30 in step S19 (step S53). The control unit 21 and the communication unit 23 correspond to an example of the "reception unit", and receive the degree information about the degree of the specific action. The control unit 21 may perform control such that the specific action information and the degree information simultaneously transmitted from the first information terminal 30 are simultaneously received.

The control unit 21 acquires at least one second content stored in association with the specific action information (more specifically, degree information), from the storage unit 22 (step S54). Subsequently, the control unit 21 transmits the second content acquired in step S54 to the first information terminal 30 (step S55). The control unit 21 and the communication unit 23 correspond to an example of the "transmission unit", and transmit at least one second content to the first information terminal 30 when it is determined that the first user has ended the specific action. For example, in the example in Fig. 4(B), each second content is associated with each menu, and in such an example, the second content associated with the menu executed in step S54 can be acquired, and the second content thus acquired can be transmitted to the first information terminal 30.

In the example in Fig. 4(B), each second content is associated with each menu, and in this example, when the specific action of any menu is performed, the second content corresponding to the menu may be acquired in step S54. Examples of the second content associated with the menu include the next menu that is recommended if that menu is performed, etc. The next menu that is recommended if the menu included in the previous first content is performed corresponds to an example of suggestion information. The suggestion information is not limited to this example. For example, if the degree of achievement of the menu included in the previous first content is within a predetermined range that is significantly lower than a target value, the suggestion information may be information that suggests a menu having a slightly lower load than the above "next menu".

The present invention is not limited to the example in Fig. 4(B). When the reception unit receives the degree information, a second content including evaluation information reflecting degree information may be adopted in step S54. The evaluation information reflecting the degree information may include, for example, information of an achievement degree that indicates the degree of achievement with respect to a predetermined target value, message information that conveys or praises achievement of the target value, etc. The above degree information or the above evaluation information may include exercise intensity. The exercise intensity may be calculated by the first information terminal 30 or by the content management server 20. For calculating the exercise intensity, for example, MET, which indicates the intensity of physical activity, may be used. In this example, in the case of an exercise such as strolling, a speed calculated from the distance and the time of strolling is an index of MET. As another example, in the case of strolling, the intensity of physical activity based on speed can be determined. For example, the exercise intensity may be calculated or detected such that: if the number of steps per minute is within 100, the exercise intensity is determined as exercise intensity 1; if the number of steps per minute exceeds 100 and is within 150, the exercise intensity is determined as exercise intensity 2 higher than the exercise intensity 1; and if the number of steps per minute exceeds 150, the exercise intensity is determined as exercise intensity 3 higher than the exercise intensity 2. As a further application example, a slope or stair in a strolling route may be calculated from an acceleration sensor included in a wearable device or information terminal, and be used for multiplying the above exercise intensity to correct the exercise intensity to exercise intensity corresponding to the slope or stair. The above second content may be a content including both the above evaluation information and the above suggestion information, or may be a content including only either one of the above evaluation information and the above suggestion information.

Subsequently, the control unit 21 generates second notification information (step S56). The second notification information is information including information indicating that the specific action has ended. Specifically, the second notification information is information that notifies that the first user has ended walking or running. Subsequently, the control unit 21 transmits the second notification information generated in step S56, to the second information terminal 40 (step S57). The control unit 21 and the communication unit 23 correspond to an example of the "transmission unit", and transmit the second notification information including information indicating that the specific action has ended, to the second information terminal 40 when it is determined that the first user has ended the specific action. After step S57, the control unit 21 ends the second content transmission control shown in Fig. 10.

### 1-9. Second content output control by first information terminal 30

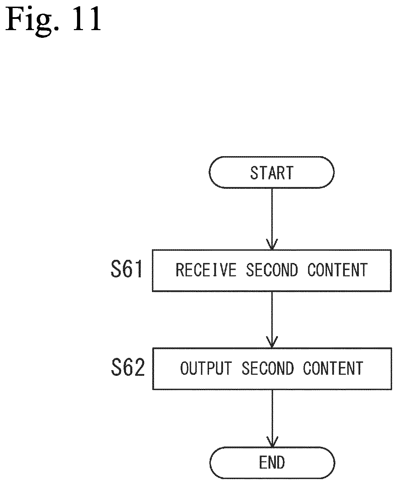

Second content output control by the first information terminal 30 will be described with reference to a flowchart shown in Fig. 11. The flowchart shown in Fig. 11 is a flowchart showing the flow of the second content output control executed by the control unit 31 of the first information terminal 30.

The control unit 31 performs the control in Fig. 11, for example, when the second content is transmitted from the content management server 20. When performing the control in Fig. 11, first, the control unit 31 receives the second content transmitted from the content management server 20 in step S55 (step S61). Subsequently, the control unit 31 outputs the second content received in step S61 (step S62). For example, the method for outputting the second content is the same as the above-described method for outputting the first content. The first user (user of the first information terminal 30) can obtain the second content after performing the specific action, and the second content is information that can increase the will to perform the specific action again, so that a motivation to perform the specific action again can be given to the first user. In addition, a different kind of second content can be given to the first user according to the degree of the specific action performed. After step S62, the control unit 31 ends the second content output control shown in Fig. 11.

### 1-10. Notification control, based on second notification information, by second information terminal 40

Notification control, based on the second notification information, by the second information terminal 40 will be described with reference to a flowchart shown in Fig. 12. The flowchart shown in Fig. 12 is a flowchart showing the flow of the notification control, based on the second notification information, executed by the control unit 41 of the second information terminal 40.

The control unit 41 performs the control in Fig. 12, for example, when the second notification information is transmitted from the content management server 20. First, the control unit 41 receives the second notification information transmitted from the content management server 20 in step S57 (step S71). Subsequently, the control unit 41 notifies the end of the specific action on the basis of the second notification information received in step S71 (step S72). The method for notifying the end of the specific action is the same as the above-described method for notifying the start of the specific action. For example, a message such as "Walking has ended" may be displayed on the display unit 44. Through the notification based on the second notification information, the second user (user of the second information terminal 40) can confirm the safety of the first user after the specific action. In addition, the second user can not only receive the second notification information (information about the safety of the first user after the specific action) but also can decide on the second content to be transmitted to the first user. Thus, the second user can not only confirm whether or not the specific action has been appropriately performed, but also can provide specific information that can enhance the continuity of the specific action, at the discretion of the second user, and furthermore, a kind of communication can be also performed. After step S72, the control unit 41 ends the notification control, based on the second notification information, shown in Fig. 12.

In the present embodiment, the vital information of the first user may be detected while the first user is performing the specific action or after the specific action has ended, and the detected vital information may be transmitted from the first information terminal 30 to the content management server 20. For example, the first information terminal 30 may acquire the vital information of the first user immediately after the specific action has ended, and may transmit the vital information to the content management server 20. In this case, as the vital information, the first information terminal 30 may acquire values measured by another measurement device (e.g., a wearable terminal worn by the first user, or the like) different from the first information terminal 30, via wireless communication, and may transmit the values to the content management server 20, or the first information terminal 30 itself may measure vital information and may transmit the measured vital information to the content management server 20. In this example, when the reception unit of the content management server 20 receives the vital information during or after the specific action of the first user, the vital information may be stored in the storage unit 22 and/or may be transmitted to the second information terminal 40. In this example, the control unit 21 and the communication unit 23 correspond to an example of a handling unit.

When the handling unit transmits the vital information to the second information terminal 40 as described above, the second user who uses the second information terminal 40 can grasp and evaluate the vital information. Then, the second user may operate the second information terminal 40 to input information including a diagnosis result and desired treatment action grasped from the vital information, as response information (response information corresponding to the vital information), and to transmit the response information from the second information terminal 40 to the content management server 20. When the response information is transmitted from the second information terminal 40 as described above, the reception unit of the content management server 20 can receive the response information, and the transmission unit of the content management server 20 can transmit the response information to the first information terminal 30. Through such operation, the first user can obtain an opinion, a diagnosis result, future treatment contents, and a treatment plan for the symptoms of the second user, evaluated on the basis of the vital information during or after the specific action, which can be used for subsequent appropriate exercise and rehabilitation, etc. By grasping the vital information in the content management server 20, the second user can use the vital information to grasp the symptoms of the first user, diagnose the first user, and determine treatment contents and a treatment plan, when the first user undergoes a medical examination.

### 1-11. Effects of first embodiment

The content management server 20 can transmit the first content, which provides at least either the menu of the specific action or the effect of the specific action as information, to the first information terminal 30 in response to the reception of the preparation action information about the preparation action before the specific action. Therefore, the management server 20 can give a motivation that easily increases the will to perform the specific action, to the first user who uses the first information terminal 30, at the stage of the preparation action before the specific action.

Since the content management server 20 can provide the menu based on the vital information of the first user before the specific action, the menu reflecting the status of the vitals of the first user before performing the specific action can be provided.

Since the content management server 20 can collect the "degree information indicating the degree of the specific action" detected by the first information terminal 30, the actual execution result of the first user can be properly grasped, and it is made possible to use the degree information later.

The content management server 20 can provide a suggestion menu reflecting the degree information acquired from the first information terminal 30 or a corresponding effect based on the degree information, to the first information terminal 30. Therefore, evaluation and suggestion based on the result of the specific action actually performed by the first user who uses the first information terminal 30 can be enabled, so that a new motivation to perform the next specific action can be more effectively given to the first user.

The content management server 20 can receive the vital information during or after the specific action of the first user, and it is made possible to use the vital information at the management server 20 or the second information terminal 40. The vital information during or after the specific action is useful information for grasping what changes occurred in the body of the first user when the specific action was performed. Therefore, the content management server 20 in [5] gives a motivation that easily increases the will of the first user, and also makes it easier to properly perform body management of the first user when the first user actually performs the specific action.

When the first user performs the specific action, the content management server 20 can provide the vital information of the first user during or after the specific action to the second user side to allow the second user to make an evaluation, and can convey the response information returned from the second user side based on the vital information, to the first user side.

The content management server 20 allows the second user who watches the first user to confirm that the first user will start the specific action, on the basis of the information indicating that the specific action is to be started. Accordingly, the second user can confirm the safety of the first user before the specific action.

The content management server 20 can receive the first content to be stored in the storage unit, from the second information terminal 40. With such a configuration, the user of the second information terminal 40 can decide on the content to be transmitted to the first user.

After the specific action, the content management server 20 can transmit the second content to the first user who uses the first information terminal 30. Accordingly, the first user can recognize that the second content can be obtained after performing the specific action, so that a motivation to perform the specific action again can be given to the first user.

The content management server 20 allows the second user who watches the first user to confirm that the first user has ended the specific action, on the basis of the information indicating that the specific action has ended. Accordingly, the second user can confirm the safety of the first user after the specific action.

The content management server 20 can receive the second content to be stored in the storage unit, from the second information terminal 40. With such a configuration, the user of the second information terminal 40 can decide on the details of the second content to be given to the first user after the specific action.

### 2. Second embodiment

### 2-1. Configuration of content management system 210

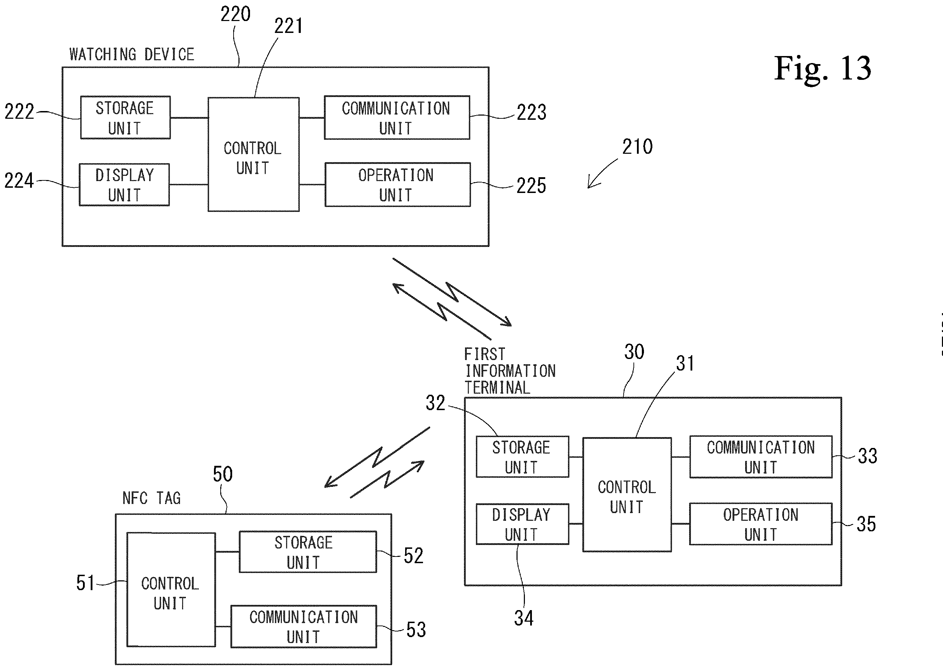

A content management system 210 shown in Fig. 13 is mainly different from the first embodiment in that a content management device that is not configured as a server is included in place of the content management server. The same components as those in the first embodiment are denoted by the same reference characters, and the detailed description thereof is omitted.

The content management system 210 shown in Fig. 13 includes a content management device 220, a first information terminal 30, and an NFC tag 50. The content management device 220 can communicate with the first information terminal 30. The content management system 210 is a system that can transmit contents stored in the content management device 220 to the first information terminal 30.

The content management device 220 is, for example, a device that is installed in the residence or the like of a first user and that is used by the first user. The content management device 220 includes a control unit 221, a storage unit 222, a communication unit 223, a display unit 224, and an operation unit 225. The control unit 221, the storage unit 222, the display unit 224, and the operation unit 225 have the same configuration as the control unit 21, the storage unit 22, the display unit 24, and the operation unit 25 of the first embodiment. The communication unit 223 is configured as a communication module that can perform wireless communication with the first information terminal 30, for example, by a short-range communication method (communication method conforming to a wireless communication standard such as Bluetooth (registered trademark)).

### 2-2. Upload of contents

In the content management device 220, contents (first and second contents) are stored in advance. For example, a storage medium having contents recorded by a watching person or the like is read by the content management device 220 so that the contents are stored in the storage unit 222 of the content management device 220. Contents recorded in the second information terminal 40 may be transferred to the content management device 220 by connecting the second information terminal 40 described in the first embodiment to the content management device 220 via a communication interface.

### 2-3. Communication process of content management system 210

Action information transmission control by the first information terminal 30 is the same as in the first embodiment (see Fig. 5). First content transmission control by the content management device 220 is generally the same as in the first embodiment (see steps S21 to S23 in Fig. 6). The content management device 220 may be configured to be able to communicate with another information terminal (second information terminal 40 or the like) via a wide-area communication network (the Internet communication network or the like) and transmit first notification information to the other information terminal by the same process as in steps S24 and S25 in Fig. 6. First content output control by the first information terminal 30 is the same as in the first embodiment (see Fig. 7).

Second content transmission control by the control unit 221 of the content management device 220 is generally the same as in the first embodiment (see steps S51 to S55 in Fig. 10). The content management device 220 may be configured to transmit second notification information to the other information terminal by the same process as in steps S56 and S57 in Fig. 10.

with the content management system 210 of the second embodiment also, a reinforcer (the first content similar to that in the first embodiment) that increases the will to perform the specific action can be given to the first user at the stage of the preparation action before the specific action by outputting the first content by the first information terminal 30. Accordingly, a motivation to perform the specific action is provided to the first user.

### 3. Third embodiment

### 3-1. Configuration of content management system 310

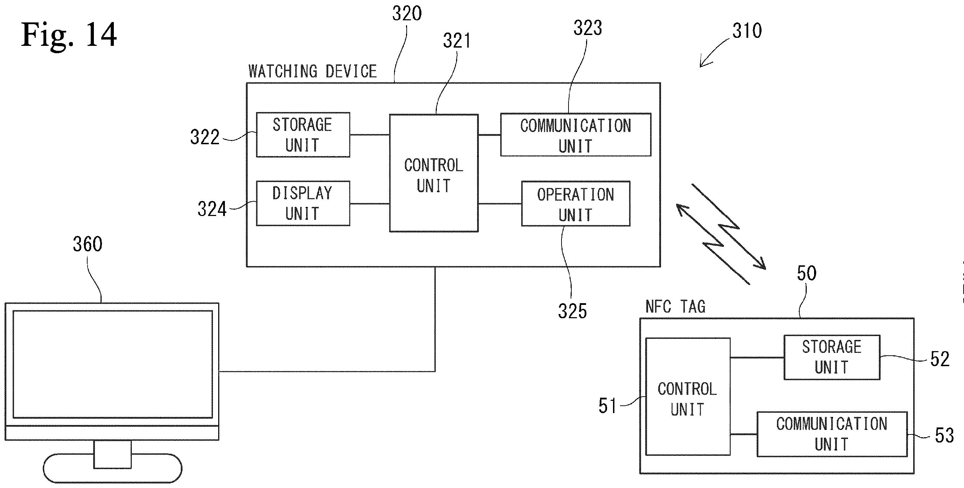

A content management system 310 shown in Fig. 14 is mainly different from the first embodiment in that a television set and a content management device that is not configured as a server are included in place of the content management server. The same components as those in the first embodiment are denoted by the same reference characters, and the detailed description thereof is omitted.

The content management system 310 shown in Fig. 14 includes a content management device 320, a television set 360, and an NFC tag 50. The content management system 310 is a system that can output contents stored in the content management device 320, from the television set 360.

The content management device 320 is configured as a so-called set-top box, and functions such that contents are outputted from the television set 360 on the basis of broadcast signals received from the outside. The content management device 320 is, for example, a device that is installed in the residence or the like of a first user and that is used by the first user. The content management device 320 includes a control unit 321, a storage unit 322, a communication unit 323, a display unit 324, and an operation unit 325. The control unit 321, the storage unit 322, the display unit 324, and the operation unit 325 have the same configuration as the control unit 21, the storage unit 22, the display unit 24, and the operation unit 25 of the first embodiment. The communication unit 323 is configured as a communication module that can perform communication with another information terminal (second information terminal 40 or the like), for example, through a wide-area communication network (the Internet communication network or the like).

### 3-2. Upload of contents

In the content management device 320, contents (first and second contents) are stored in advance. For example, the content management device 320 is configured to receive contents from the other information terminal (second information terminal 40 or the like) through the wide-area communication network (the Internet communication network or the like). As in the second embodiment, contents may be directly stored in the storage unit 322 of the content management device 320 using a storage medium or the like.

### 3-3. Communication process of content management system 310

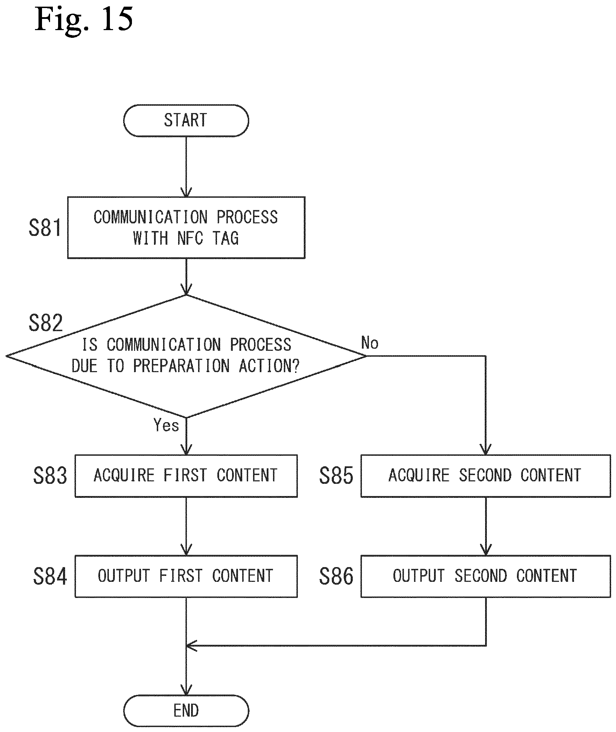

Content output control by the content management device 320 will be described with reference to a flowchart shown in Fig. 15. The flowchart shown in Fig. 15 is a flowchart showing the flow of the content output control performed by the control unit 321 of the content management device 320.

First, as in step S11 in Fig. 5, the control unit 321 performs a communication process with the NFC tag 50 (step S81). Subsequently, as in step S12 in Fig. 5, the control unit 321 determines whether or not the communication process performed in step S81 is due to the preparation action before the specific action (step S82). Here, the control unit 321 corresponds to an example of a "detection unit" of the present invention, and detects the preparation action. For example, the control unit 321 is configured to repeatedly determine, every time a communication process with the NFC tag 50 is performed, that it is a communication process before the specific action, a communication process after the specific action, a communication process before the specific action ..., so that the control unit 321 determines that the present communication process is a communication process before the specific action when the previous communication process is a communication process after the specific action. Alternatively, the control unit 321 may determine that the present communication process is a communication process before the specific action when the elapsed time from the previous communication process has exceeded a certain time (12 hours or the like).

When the determination in step S82 is Yes, the control unit 321 acquires the first content from the storage unit 322 (step S83). Subsequently, the control unit 321 outputs the first content acquired in step S83, from the television set 360 (step S84). The control unit 321 corresponds to an example of an "output unit" of the present invention. The output method can be the same as in step S32 in Fig. 7 described above. After the process in step S84, the control unit 321 ends the content output control shown in Fig. 15.

On the other hand, when the determination in step S82 is No, the control unit 321 acquires the second content from the storage unit 322 (step S85). Subsequently, the control unit 321 outputs the second content acquired in step S85, from the television set 360 (step S86). The output method can be the same as in step S34. After the process in step S86, the control unit 321 ends the content output control shown in Fig. 15.

The content management device 320 may be configured to be able to communicate with another information terminal (second information terminal 40 or the like) via the wide-area communication network (the Internet communication network or the like) and transmit first notification information to the other information terminal by the same process as in steps S24 and S25 in Fig. 6. In addition, the content management device 320 may be configured to transmit second notification information to the other information terminal as in steps S56 and S57 in Fig. 10.

In the content management system 310 of the third embodiment, the content management device 320 can output the first content similar to that in the first embodiment, in response to the detection of the preparation action before the specific action. Therefore, a reinforcer that increases the will to perform the specific action can be given to the first user at the stage of the preparation action before the specific action. Accordingly, a motivation to perform the specific action can be given to the first user.

### 4. Fourth embodiment

### 4-1. Configuration of content management system 410

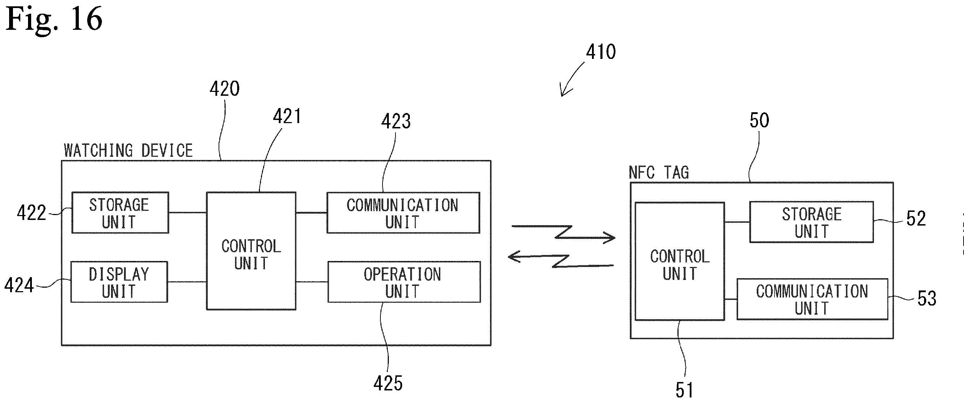

A content management system 410 shown in Fig. 16 is mainly different from the third embodiment in that a content management device 420 is included in place of the content management device 320 and the television set 360. The same components as those in the third embodiment are denoted by the same reference characters, and the detailed description thereof is omitted.

The content management system 410 shown in Fig. 16 includes the content management device 420 and an NFC tag 50. The content management device 420 is configured as a device with a monitor. The content management device 420 may be, for example, a monitor device itself, which is mainly composed of a monitor, or may be a device composed of another device (home appliance or the like) and a monitor attached thereto. The content management system 410 is a system that can output contents stored in the content management device 420.

The content management device 420 is, for example, a device that is installed in the residence or the like of a first user and that is used by the first user. The content management device 420 includes a control unit 421, a storage unit 422, a communication unit 423, a display unit 424, and an operation unit 425. The control unit 421, the storage unit 422, the display unit 424, and the operation unit 425 have the same configuration as the control unit 21, the storage unit 22, the display unit 24, and the operation unit 25 of the first embodiment. The communication unit 423 has the same configuration as the communication unit 323 of the third embodiment. The display unit 424 is configured as an image display device (monitor) such as a liquid crystal display or an organic electroluminescent display.

### 4-2. Upload of contents

In the content management device 420, contents (first and second contents) are stored in advance in the same method as in the third embodiment.

### 4-3. Communication process of content management system 410

Content output control by the control unit 421 of the content management device 420 is performed by the same process as in steps S81 to S86 in Fig. 15 of the third embodiment. The control unit 421 corresponds to an example of the "detection unit" of the present invention, and detects the preparation action. The control unit 421 and the display unit 424 correspond to an example of the "output unit" of the present invention. The control unit 421 causes the first and second contents to be outputted by the display unit 424. The output method can be the same as in step S34 in Fig. 7 of the first embodiment.

In the content management system 410 of the fourth embodiment, the content management device 420 can output the first content similar to that in the first embodiment, in response to the detection of the preparation action before the specific action. Therefore, a reinforcer that increases the will to perform the specific action can be given to the first user at the stage of the preparation action before the specific action. Accordingly, a motivation to perform the specific action can be given to the first user.

### 5. Fifth embodiment

### 5-1. Configuration of content management system 510

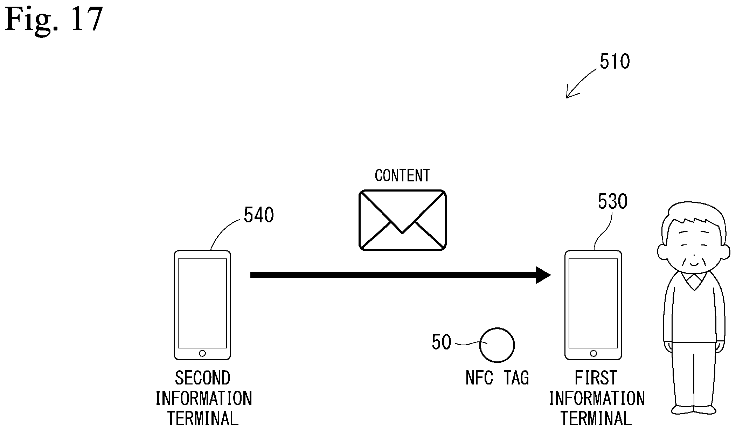

A content management system 510 shown in Fig. 17 is mainly different from the fourth embodiment in that a content management device is configured as an information terminal. The same components as those in the first to fourth embodiments are denoted by the same reference characters, and the detailed description thereof is omitted.

The content management system 510 shown in Fig. 17 includes a first information terminal 530, a second information terminal 540, and an NFC tag 50. The first information terminal 530 corresponds to an example of the "content management device" of the present invention. The first information terminal 530 has the same configuration as the first information terminal 30 of the first embodiment. The first information terminal 530 is used by a first user who is a target to be watched. The first information terminal 530 outputs contents to the first user. The second information terminal 540 is used by a second user who watches the first user. The content management system 510 is a system that can output a content transmitted from the second information terminal 540, by the first information terminal 530.

The contents (first and second contents) have the same configuration as the contents (first and second contents) used in the first embodiment. The contents are reinforcers (rewards) that increase the will of the first user to perform the specific action, at the stage of the preparation action before the specific action.

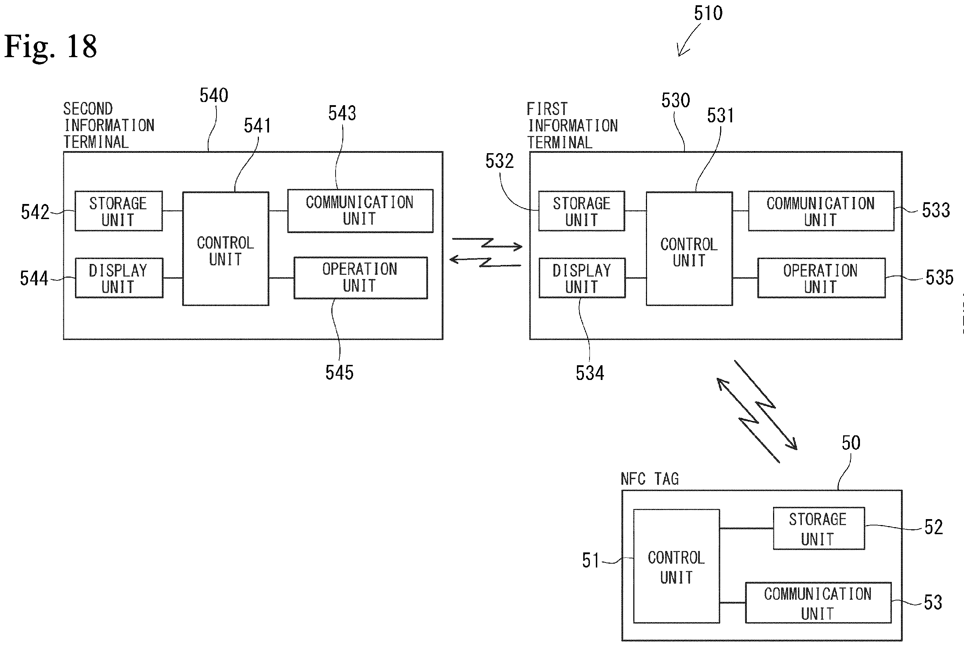

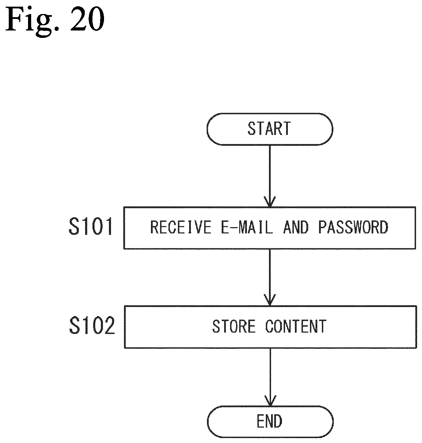

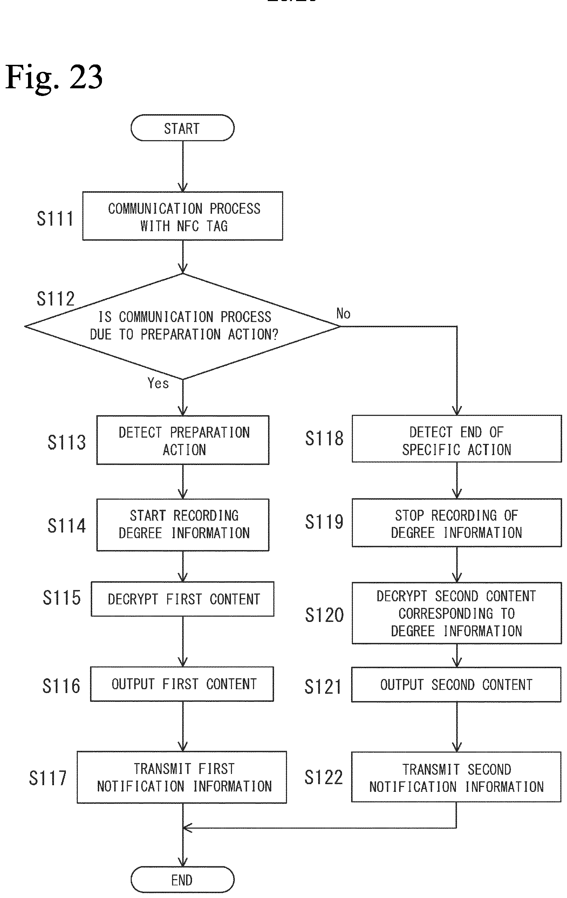

As shown in Fig. 18, the configuration of the first information terminal 530 is the same as that of the first information terminal 30 of the first embodiment. The first information terminal 530 has a control unit 531, a storage unit 532, a communication unit 533, a display unit 534, and an operation unit 535 which have the same configuration as the control unit 31, the storage unit 32, the communication unit 33, the display unit 34, and the operation unit 35 of the first information terminal 30 of the first embodiment, respectively. The control unit 531 and the display unit 534 correspond to an example of the "output unit" of the present invention. The control unit 531 causes the first and second contents to be outputted by the display unit 534. In the first information terminal 530, an application program (hereinafter, also referred to as management application) for storing and outputting contents, etc., is installed. The management application is a program that allows control described later (see Fig. 20 and Fig. 23) to be executed. The management application can cooperate with a mailer (application program for sending and receiving e-mails, etc.) installed in the first information terminal 530. By cooperating with the mailer, the management application can, for example, set access rights (access permissions) to a mail server and allow e-mails to be sent and received.

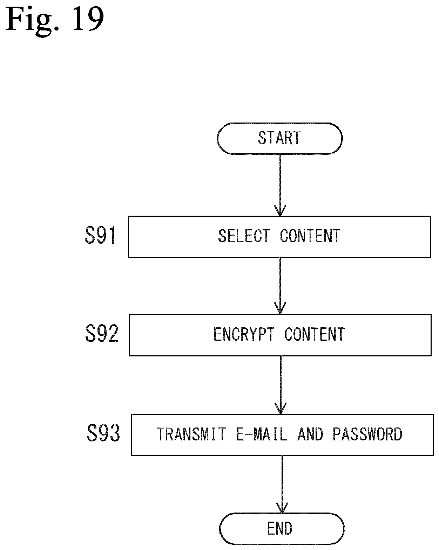

The configuration of the second information terminal 540 is the same as that of the second information terminal 40 of the first embodiment. The second information terminal 540 has a control unit 541, a storage unit 542, a communication unit 543, a display unit 544, and an operation unit 545 which have the same configuration as the control unit 41, the storage unit 42, the communication unit 43, the display unit 44, and the operation unit 45 of the second information terminal 40 of the first embodiment, respectively. In the second information terminal 540, an application program (hereinafter, also referred to as management application) for storing and transmitting contents, etc., is installed. The management application is a program that allows control described later (see Fig. 19) to be executed. The management application can cooperate with a mailer (application program for sending and receiving e-mails, etc.) installed in the second information terminal 540. By cooperating with the mailer, the management application can, for example, set access rights (access permissions) to a mail server and allow e-mails to be sent and received.

### 5-2. Outline of operation of content management system

Fig. 17 is an explanatory diagram illustrating an outline of operation of the content management system when contents (e.g., first and second contents) are transmitted from the second information terminal 540 to the first information terminal 530. The contents are transmitted from the second information terminal 540 to the first information terminal 530 in a state where access to the contents is restricted. The first information terminal 530 stores therein the contents in a state where access to the contents is restricted.

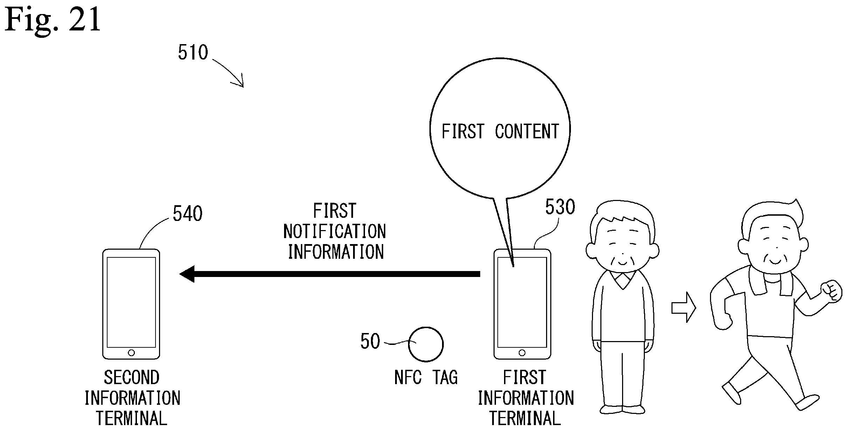

Fig. 21 is an explanatory diagram illustrating an outline of operation of the content management system before the specific action. When the preparation action before the specific action is detected by the first information terminal 530, the first content is outputted from the first information terminal 530. In addition, when the preparation action is detected by the first information terminal 530, first notification information described later is transmitted from the first information terminal 530 to the second information terminal 540.

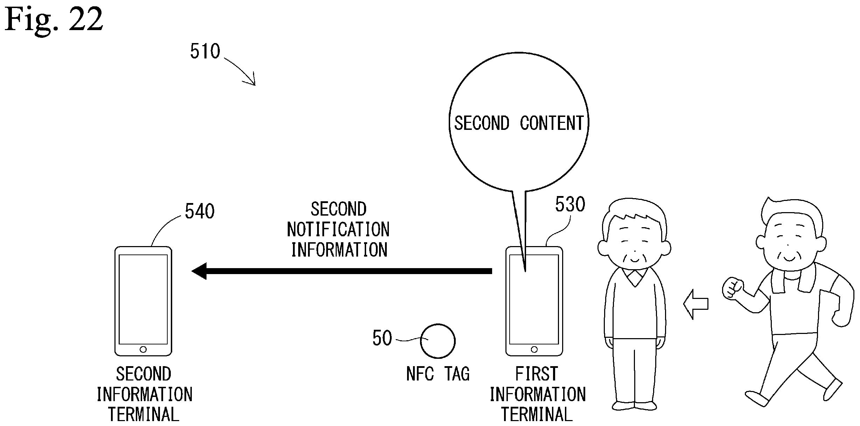

Fig. 22 is an explanatory diagram illustrating an outline of operation of the content management system after the specific action. When the end of the specific action is detected by the first information terminal 530, the second content is outputted by the first information terminal 530. In addition, when the end of the specific action is detected by the first information terminal 530, second notification information described later is transmitted from the first information terminal 530 to the second information terminal 540.

### 5-3. Content transmission control by second information terminal 540

Content transmission control by the second information terminal 540 will be described with reference to a flowchart shown in Fig. 19. The flowchart shown in Fig. 19 is a flowchart showing the flow of the content transmission control executed by the control unit 541 of the second information terminal 540.

First, the control unit 541 performs a process of selecting a content (first content or second content) (step S91). For example, the control unit 541 selects a content stored in the second information terminal 540 (e.g., the storage unit 542) on the basis of an operation by the second user on the operation unit 545 or the like. Here, as for the second content, a plurality of kinds of second contents may be selected in association with degree information about degrees of the specific action (degree information having the same configuration as in the first embodiment). Subsequently, the control unit 541 encrypts the content (step S92). For example, the control unit 541 sets a password (encryption key) and encrypts the content on the basis of the password.

Subsequently, the control unit 541 transmits the encrypted content and the password to the first information terminal 530 using the mailer (step S93). For example, the control unit 541 transmits an e-mail having the encrypted content and the password attached thereto, to the first information terminal 530 under control of the management application which cooperates with the mailer.

The encrypted content and the password are transmitted by an e-mail in association with each other. For example, the encrypted content and the password are transmitted in association with a common identification number or the like. The number of contents attached to one e-mail may be one or a plural number. As for the content attached to the e-mail, the kind of the content (first content or second content) is identifiable. When transmitting an e-mail, the control unit 541 may add an identifier (a character string or the like) to the title or body of the e-mail. After step S93, the control unit 541 ends the content transmission control shown in Fig. 19.

### 5-4. Content reception control by first information terminal 530

Content reception control by the first information terminal 530 will be described with reference to a flowchart shown in Fig. 20. The flowchart shown in Fig. 20 is a flowchart showing the flow of the content reception control executed by the control unit 531 of the first information terminal 530.

First, the control unit 531 receives the e-mail having the encrypted content (first content or second content) and the password attached thereto, from the second information terminal 540 (step S101). The control unit 531 and the communication unit 533 correspond to an example of the "reception unit" of the present invention, and receives the content from the second information terminal 540.

Subsequently, the control unit 531 stores the encrypted content and the password attached to the e-mail received in step S101, in the storage unit 532. The storage unit 532 stores therein the content in a state where access to the content is restricted. Restricting access to the content means disabling output of the content and restricting viewing of the content. The encrypted content and the password attached to the e-mail received in step S101 are stored in the storage unit 532. The encrypted content and the password are stored in the storage unit 532 in association with each other. For example, the encrypted content and the password are received in association with a common identification number or the like. Here, as for the second content, a plurality of kinds of second contents may be stored in association with degrees of the specific action (degree information).

When an identifier (e.g., a character string) is added to the title or body of an e-mail to be received, the identifier makes it easier to specify the e-mail as an e-mail having a content attached thereto. The encrypted content and the password may be automatically stored in the storage unit 532 under control of the management application, or may be stored therein on the basis of an operation by the first user on the operation unit 545. After step S102, the control unit 531 ends the content reception control shown in Fig. 20.

### 5-5. Content output control by first information terminal 530

Content output control by the first information terminal 530 will be described with reference to a flowchart shown in Fig. 23. The flowchart shown in Fig. 23 is a flowchart showing the flow of the content output control executed by the control unit 531 of the first information terminal 530.

First, as in step S11 in Fig. 5, the control unit 531 performs a communication process with the NFC tag 50 (step S111). Subsequently, as in step S12 in Fig. 5, the control unit 531 determines whether or not the communication process performed in step S111 is due to the preparation action before the specific action (step S112). Here, the control unit 531 corresponds to an example of the "detection unit" of the present invention, and detects the preparation action before the specific action. For example, the control unit 531 is configured to repeatedly determine, every time a communication process with the NFC tag 50 is performed, that it is a communication process before the specific action, a communication process after the specific action, a communication process before the specific action ..., so that the control unit 531 determines that the present communication process is a communication process before the specific action when the previous communication process is a communication process after the specific action,. Alternatively, the control unit 531 may determine that the present communication process is a communication process before the specific action when the elapsed time from the previous communication process has exceeded a certain time (12 hours or the like).

When the determination in step S112 is Yes, the control unit 531 determines that the preparation action has been detected (step S113). Subsequently, the control unit 531 detects the degree of the specific action, and starts recording data (degree information) of the specific action as in step S15 in Fig. 5 (step S114). Subsequently, the control unit 531 performs a process of decrypting the first content (step S115). For example, the control unit 531 performs decryption using the password associated with the first content. Subsequently, the control unit 531 performs a process of outputting the first content (display of a photograph or a moving image by the display unit 534, output of a sound by a speaker or the like) as in step S32 in Fig. 7 (step S116). As described above, the control unit 531 cancels the restriction of access to the first content and outputs the first content. By outputting the first content by the first information terminal 530, a reinforcer (first content) that increases the will of the first user to perform the specific action, at the stage of the preparation action before the specific action, can be given to the first user at the stage of the preparation action before the specific action. Accordingly, a motivation to perform the specific action can be given to the first user. In addition, since the specific action of the first user is immediately reinforced, the specific action can be continued and a habit of the specific action can be acquired. If the specific action is exercise, a health promotion effect can be obtained.

Subsequently, the control unit 531 generates first notification information and transmits the first notification information to the second information terminal 540 (step S117). The control unit 531 and the communication unit 533 correspond to an example of the "transmission unit" of the present invention, and transmit first notification information including information indicating that the specific action is to be started, to the second information terminal 540 when the preparation action is detected. The first notification information is the same as the first notification information generated in step S24 in Fig. 6. Through the notification based on the first notification information, the second user (user of the second information terminal 40) can confirm the safety of the first user before the specific action. In addition, the second user can not only receive the first notification information (information about the safety of the first user before the specific action) but also can decide on the first content to be transmitted to the first user, and thus a kind of communication can be performed. Communication between the first user and the second user can be promoted by the events or topic derived from the first content. After step S117, the control unit 531 ends the content output control shown in Fig. 23.

On the other hand, when the control unit 531 determines in step S112 that the communication process performed in step S111 is not due to the preparation action before the specific action (that is, due to an action after the specific action), the control unit 531 proceeds to No and determines that the end of the specific action has been detected (step S118). Whether or not the communication process was performed by the first information terminal 530 and the NFC tag 50 after the specific action was performed can be determined by the same method as in step S12 in Fig. 5.

Subsequently, the control unit 531 stops the recording of the degree information of the specific action that has been started in step S114 previously performed (step S119). Subsequently, the control unit 531 performs a process of decrypting the second content corresponding to the degree information (step S120). For example, the control unit 531 performs decryption using the password associated with the second content. The second content stored in association with the degree information can be acquired by the same method as in step S54 in Fig. 10. Subsequently, as in step S62 in Fig. 11, the control unit 531 performs a process of outputting at least one second content (display of a photograph or a moving image by the display unit 534, output of a sound by a speaker or the like) (step S121). As described above, the control unit 531 cancels the restriction of access to the second content and outputs the second content. The first user (user of the first information terminal 530) can recognize that the second content can be obtained after performing the specific action, so that a motivation to perform the specific action again can be given to the first user. In addition, a different kind of second content can be given to the first user according to the degree of the specific action performed.

Subsequently, the control unit 531 generates second notification information and transmits the second notification information to the second information terminal 540 (step S122). The control unit 531 and the communication unit 533 correspond to an example of the "transmission unit" of the present invention, and transmit second notification information including information indicating that the specific action has ended, to the second information terminal 540 when the end of the specific action is detected. The second notification information is the same as the second notification information generated in step S56 in Fig. 10. Through the notification based on the second notification information, the second user (user of the second information terminal 540) can confirm the safety of the first user after the specific action. In addition, the second user can not only receive the second notification information (information about the safety of the first user after the specific action) but also can decide on the second content to be transmitted to the first user, and thus a kind of communication can be performed. After step S122, the control unit 531 ends the content output control shown in Fig. 23.

### 5-6. Effects of fifth embodiment

The content management system 510 does not require a management server or the like for managing contents, and can give a reinforcer to the first user using the contents stored in advance in the first information terminal 530. Therefore, it is not necessary to set up and maintain a dedicated server as a management server, and the system can be constructed with an inexpensive configuration.

In the content management system 510, the storage unit 532 of the first information terminal 530 stores therein the first content in a state where access to the first content is restricted. When the preparation action is detected, the control unit 531 and the display unit 534 cancel the restriction of access to the first content and output the first content. With this configuration, it is possible to prevent the first user who uses the first information terminal 530 from accessing the first content before performing the preparation action. Thus, the first content is allowed to be accessed for the first time by performing the preparation action, so that a reinforcer can be reliably given at the stage of the preparation action before the specific action.

In the content management system 510, the storage unit 532 of the first information terminal 530 stores therein the second content in a state where access to the second content is restricted. When it is determined that the first user has ended the specific action, the control unit 531 and the display unit 534 cancel the restriction of access to the second content and output the second content. With this configuration, it is possible to prevent the first user who uses the first information terminal 530 from accessing the second content before ending the specific action. Thus, the second content is allowed to be accessed for the first time by ending the specific action, so that a reinforcer can be reliably given when the specific action is ended.

### 6. Other embodiments

The present invention is not limited to the embodiments described by the above description and the drawings, and, for example, the following embodiments are also included in the technical scope of the present invention. In addition, various features of the above-described embodiments and the embodiments described below may be combined in any manner as long as the combination is not contradictory.

In the above first and second embodiments, the control unit 31 of the first information terminal 30 determines whether or not the preparation action has been detected, on the basis of the presence/absence of a communication process with the NFC tag 50, but may determine whether or not the preparation action has been detected, by another method. For example, a start button or the like may be displayed on the screen of the first information terminal 30 when the management application is launched, and whether or not the preparation action has been detected may be determined on the basis of the presence/absence of an operation of pressing the start button or the like. Alternatively, a camera may be provided at an arbitrary location (e.g., entrance) in the residence of the first user, and whether or not the preparation action has been detected may be determined on the basis of whether or not the first user is captured by the camera. Still alternatively, a GPS device may be installed in the first information terminal 30, and whether or not the preparation action has been detected may be determined by determining whether or not the position of the first information terminal 30 is at a predetermined location (e.g., entrance), on the basis of position information of the first information terminal 30.

In the third and fourth embodiments, the control unit of the content management device determines whether or not the preparation action has been detected, on the basis of the presence/absence of a communication process with the NFC tag 50, but may determine whether or not the preparation action has been detected, by another method. For example, whether or not the preparation action has been detected may be determined on the basis of the presence/absence of an operation of pressing a start button which is additionally provided to the content management device.

In the first to fifth embodiments, whether or not the preparation action has been detected is determined on the basis of the presence/absence of a communication process with the NFC tag 50, but may be determined on the basis of the presence/absence of a communication process with another tag or device. Whether or not the preparation action has been detected may be determined, for example, on the basis of the presence/absence of a communication process with another tag or device according to the Bluetooth (registered trademark) communication standard.

In the first embodiment, whether or not the communication process with the NFC tag 50 is due to the preparation action is determined by the control unit 31 of the first information terminal 30. However, the content management server 20, after receiving degree information, may determine whether or not the communication process with the NFC tag 50 is due to the preparation action (whether it is the start of the specific action or the end thereof), by the same method as in step S12 in Fig. 5. The content management device 220 of the second embodiment may have the same configuration.

In the first and second embodiments, in the determination as to whether or not the communication process is due to the preparation action before the specific action (step S12), it is determined that the specific action has been performed when the degree information is equal to or greater than a certain degree (e.g., a movement distance or the number of steps taken is equal to or greater than a certain degree). However, the determination may be made by the same method as in the third and fourth embodiments (method based on the previous communication process).

In the first to fifth embodiments, for the determination as to whether or not the communication process is due to the preparation action before the specific action (e.g., step S12), two types of start buttons may be provided to the first information terminal or the content management device such that the buttons correspond to before and after the specific action, respectively (the buttons may be in the display screen of the management application), and the first user may selectively perform a pressing operation to select either before or after the specific action.

In the first and second embodiments, when the content management server 20 receives the preparation action information, the content management server 20 determines that the specific action has ended. However, without using the specific action information, the content management server 20 may determine that the specific action has ended on the basis of the degree information received from the first information terminal 30.

In the first embodiment, the content management server 20 may not necessarily transmit the second content when the degree of the specific action specified by the degree information does not reach a certain degree (e.g., a certain distance (1 km or the like), a certain number of steps taken (500 steps or the like), etc.). The content management devices of the first to fourth embodiments may have the same configuration.

In the first to fifth embodiments, the distance is exemplified as the degree of the specific action stored in association with the second content (see Fig. 4), but such a degree may be another degree (number of steps taken, or the like).

In the fifth embodiment, the first information terminal 530 encrypts the content and stores therein the content in a state where access to the content is restricted, but access to the content may be restricted by another method. For example, access to the content may be restricted under control of the management application without encrypting the content. Meanwhile, the access restriction means that the first user is restricted from viewing or listening to a photograph, a moving image, a sound, or the like, which are the details of the content, without performing the preparation action, and does not prevent the first user from deleting or moving files that are stored or temporarily saved in the first information terminal.

In the fifth embodiment, as the method for transmitting a content from the second information terminal 540 to the first information terminal 530, the method of attaching the content to an e-mail and sending the e-mail is exemplified, but another method may be used. For example, the second information terminal 540 may upload the content to a server capable of communication, and the server may cause the first information terminal 530 to download the content therefrom. For example, the timing when the first information terminal 530 downloads the content is not particularly limited. For example, the first information terminal 530 may automatically download the content from the server when performing WiFi (registered trademark) communication. The first information terminal 530 may store the content in a state where access to the content is restricted.

Also, for example, the content may be transmitted from the second information terminal 540 to the first information terminal 530 using a communication method conforming to a wireless communication standard such as Bluetooth (registered trademark).

The embodiments disclosed herein are merely illustrative in all aspects and should not be recognized as being restrictive. The scope of the present invention is not limited to the embodiments disclosed herein, and is intended to include all modifications within the scope defined by the claims or the scope of the equivalents to the claims.

### DESCRIPTION OF REFERENCE NUMERALS

10: content management system
20: content management server
21: control unit (reception unit, transmission unit, server-side reception unit, server-side transmission unit)
22: storage unit
23: communication unit (reception unit, transmission unit, server-side reception unit, server-side transmission unit)
30: first information terminal
31: control unit (first terminal-side transmission unit, first terminal-side reception unit)
33: communication unit (first terminal-side transmission unit, first terminal-side reception unit)
34: display unit (first terminal-side output unit)
40: second information terminal
41: control unit (second terminal-side reception unit, second terminal-side notification unit)
43: communication unit (second terminal-side reception unit)
44: display unit (second terminal-side notification unit)
50: NFC tag (wireless tag)
210: content management system
220: content management device
221: control unit (reception unit, transmission unit)
222: storage unit
223: communication unit (reception unit, transmission unit)
310: content management system
320: content management device
321: control unit (detection unit, output unit)
322: storage unit
360: television set (other device)
410: content management system
420: content management device
421: control unit (detection unit, output unit)
422: storage unit
424: display unit (output unit)
510: content management system
530: first information terminal (content management device)
531: control unit (output unit, reception unit, detection unit, transmission unit)
532: storage unit
533: communication unit (reception unit, transmission unit)
534: display unit (output unit)
535: operation unit
540: second information terminal
541: control unit
542: storage unit
543: communication unit
544: display unit
545: operation unit

## Claims

1. A content management server for transmitting a content to a first information terminal used by a first user, the content management server comprising:
a storage unit configured to store therein a first content that provides at least either a menu of a specific action or an effect of the specific action as information;
a reception unit configured to receive preparation action information about a preparation action before the specific action, from the first information terminal; and
a transmission unit configured to transmit the first content to the first information terminal in response to reception of the preparation action information.

2. The content management server according to claim 1, further comprising a determination unit in which a selection method for selecting the menu on the basis of vital information is defined in advance and which is configured to, when the reception unit receives vital information before the specific action of the first user, determine the menu on the basis of the vital information of the first user, wherein
the transmission unit transmits the first content including the menu determined by the determination unit, to the first information terminal.

3. The content management server according to claim 1 or 2, wherein, when the first information terminal transmits degree information indicating a degree of the specific action detected by the first information terminal, the reception unit receives the degree information.

4. The content management server according to claim 3, wherein, when the reception unit receives the degree information, the transmission unit transmits evaluation information or suggestion information reflecting the degree information, to the first information terminal.

5. The content management server according to claim 1, further comprising a handling unit configured to, when the reception unit receives vital information during or after the specific action of the first user, store the vital information in the storage unit and/or transmit the vital information to a second information terminal used by a second user who watches the first user.

6. The content management server according to claim 5, wherein
the handling unit transmits the vital information to the second information terminal, and
when response information corresponding to the vital information is transmitted from the second information terminal and the reception unit receives the response information, the transmission unit transmits the response information to the first information terminal.

7. The content management server according to claim 1, wherein, when the preparation action information is received, the transmission unit transmits first notification information including information indicating that the specific action is to be started, to a second information terminal used by a second user who watches the first user.

8. The content management server according to claim 1 or 7, wherein the reception unit receives the first content to be stored in the storage unit, from the second information terminal used by a second user who watches the first user.

9. The content management server according to claim 1 or 7, wherein
the storage unit stores therein a second content in association with specific action information about the specific action, and
when it is determined that the first user has ended the specific action, the transmission unit transmits the second content to the first information terminal.

10. The content management server according to claim 1 or 7, wherein, when it is determined that the first user has ended the specific action, the transmission unit transmits second notification information including information indicating that the specific action has ended, to the second information terminal used by a second user who watches the first user.

11. The content management server according to claim 9, wherein the reception unit receives the second content to be stored in the storage unit, from the second information terminal used by a second user who watches the first user.

12. A content management device for transmitting a content to a first information terminal used by a first user, the content management device comprising:
a storage unit configured to store therein a first content that provides at least either a menu of a specific action or an effect of the specific action as information;
a reception unit configured to receive preparation action information about a preparation action before the specific action, from the first information terminal; and
a transmission unit configured to transmit the first content to the first information terminal in response to reception of the preparation action information.

13. A content management device for outputting a content to a first user, the content management device comprising:
a storage unit configured to store therein a first content that provides at least either a menu of a specific action or an effect of the specific action as information;
a detection unit configured to detect a preparation action before the specific action; and
an output unit configured to output the first content directly or via another device when the preparation action is detected by the detection unit.

14. A content management system comprising: a first information terminal used by a first user; and a content management server configured to transmit a content to the first information terminal, wherein
the content management server includes
a storage unit configured to store therein a first content that provides at least either a menu of a specific action or an effect of the specific action as information,
a server-side reception unit configured to receive preparation action information about a preparation action before the specific action, from the first information terminal, and
a server-side transmission unit configured to transmit the first content to the first information terminal in response to reception of the preparation action information, and
the first information terminal includes
a first terminal-side transmission unit configured to transmit the preparation action information to the content management server,
a first terminal-side reception unit configured to receive the first content from the content management server, and
a first terminal-side output unit configured to output the first content received by the first terminal-side reception unit.

15. A content management program installed in a content management server configured to transmit a content to a first information terminal used by a first user, the content management program causing a computer to:
store therein a first content that provides at least either a menu of a specific action or an effect of the specific action as information;
receive preparation action information about a preparation action before the specific action, from the first information terminal; and
transmit the first content to the first information terminal in response to reception of the preparation action information.

16. A content management program installed in a first information terminal used by a first user, the content management program causing a computer to:
transmit preparation action information about a preparation action before a specific action of the first user;
receive a first content that is associated with the preparation action information and that provides at least either a menu of a specific action or an effect of the specific action as information; and
output the received first content.
